(19) Europäisches Patentamt
European Patent Office
Office européen des brevets

(11) EP 3 236 966 B1

(12) **EUROPEAN PATENT SPECIFICATION**

(45) Date of publication and mention of the grant of the patent:
**12.08.2020 Bulletin 2020/33**

(21) Application number: **15874331.0**

(22) Date of filing: **22.12.2015**

(51) Int Cl.:
***A61K 31/506*** *(2006.01)* ***A61K 31/428*** *(2006.01)*
***A61K 31/337*** *(2006.01)* ***A61K 45/06*** *(2006.01)*
***A61P 35/00*** *(2006.01)* ***A61K 31/505*** *(2006.01)*

(86) International application number:
**PCT/US2015/067462**

(87) International publication number:
**WO 2016/106359 (30.06.2016 Gazette 2016/26)**

(54) **COMBINATION OF RAF INHIBITORS AND TAXANES**

KOMBINATION AUS RAF-HEMMERN UND TAXANEN

COMBINAISON D'INHIBITEURS DE RAF ET DE TAXANES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.12.2014 US 201462096020 P**
**16.10.2015 US 201562242629 P**

(43) Date of publication of application:
**01.11.2017 Bulletin 2017/44**

(73) Proprietor: **DOT Therapeutics-1, Inc.**
**Menlo Park, CA 94025 (US)**

(72) Inventors:
- **BOZON, Viviana**
  **West Newton, MA 02465 (US)**
- **GALVIN, Katherine M.**
  **Newton, MA 02459 (US)**
- **BRAKE, Rachael L.**
  **Natick, MA 01760 (US)**
- **XU, Qunli**
  **Wellesley, MA 02481 (US)**
- **KANNAN, Karuppiah**
  **Newton, MA 02465 (US)**

(74) Representative: **Harris, Jennifer Lucy**
**Kilburn & Strode LLP**
**Lacon London**
**84 Theobalds Road**
**London WC1X 8NL (GB)**

(56) References cited:
**WO-A1-2009/018238 WO-A1-2010/064722**
**WO-A1-2013/144923 WO-A1-2015/148828**
**WO-A2-2009/006389 US-A1- 2010 183 600**
**US-A1- 2012 214 828**

- **"A Phase 1B Study of MLN2480 in Combination With MLN0128 or Alisertib, or Paclitaxel, or Cetuximab, or Irinotecan in Adult Participants With Advanced Nonhematologic Malignancies", ClinicalTrials.gov , 30 December 2014 (2014-12-30), XP002784013, Retrieved from the Internet: URL:https://clinicaltrials.gov/ct2/show/NCT02327169 [retrieved on 2018-08-20]**
- **BALANI S K ET AL: "Drug drug interaction predictions for MLN2480, an investigational pan-RAF inhibitor, based on nonclinical data", EUROPEAN JOURNAL OF CANCER, vol. 50, no. Suppl. 6, November 2014 (2014-11), pages 54-55, XP002784014, & 26TH EORTC-NCI-AACR SYMPOSIUM ON MOLECULAR TARGETS AND CANCER THERAPEUTICS; BARCELONA, SPAIN; NOVEMBER 18 -21, 2014 ISSN: 0959-8049**
- **MASANORI OKANIWA ET AL: "Discovery of a Selective Kinase Inhibitor (TAK-632) Targeting Pan-RAF Inhibition: Design, Synthesis, and Biological Evaluation of C -7-Substituted 1,3-Benzothiazole Derivatives", JOURNAL OF MEDICINAL CHEMISTRY, vol. 56, no. 16, 1 August 2013 (2013-08-01), pages 6478-6494, XP055500984, ISSN: 0022-2623, DOI: 10.1021/jm400778d**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

**Description**

**RELATED APPLICATIONS**

The present application claims priority from U.S. provisional patent application no. 62/096,064, filed December 23, 2014 and U.S. provisional patent application no. 62/242,629, filed October 16, 2015.

This disclosure relates to methods for the treatment of cancer. In particular, the disclosure provides methods for treatment of cancer by administering Raf inhibitors in combination with a taxane.

In 2012, there were an estimated 14.1 million cancer cases around the world. This number is expected to increase to 24 million by 2035. Cancer remains the second most common cause of death in the US, accounting for nearly 1 of every 4 deaths. In 2014, there will be an estimated 1,665,540 new cancer cases diagnosed and 585,720 cancer deaths in the US. Although medical advances have improved cancer survival rates, there is a continuing need for new and more effective treatment.

Cancer is characterized by uncontrolled cell reproduction. Antimitotic agents and antimicrotubule agents have been explored as targets for cancer therapy because of their important role in the cell division cycle. Inhibition of the mitotic machinery results in a diverse array of outcomes, primarily leading to cell death or arrest.

As the effect of antimitotic agents is not limited to cancer cells alone, the dose-limiting toxicities of these drugs in a clinical setting frequently manifest in rapidly dividing tissue and in the case of antimicrotubule agents are often accompanied by severe peripheral neuropathy in the case of antimicrotubule agents. Traditional antimitotic agents include those that directly interfere with microtubule dynamics, essential for mitotic spindle assembly and the subsequent alignment and segregation of DNA to daughter cells. Antimicrotubule agents, such as taxanes are currently being used in clinical setting. For example, paclitaxel and docetaxel have a similar spectrum of clinical activity including ovarian, lung, breast, bladder, and prostate cancers.

Taxanes stabilize microtubules by altering the kinetics of microtubule depolymerization. In mammalian cells grown in culture, high concentrations of paclitaxel cause the stabilization of aggregated microtubules. At lower concentrations that resemble exposures achieved in clinical settings, the primary effect of paclitaxel is to stabilize microtubules, and thereby dampen the dynamic instability of microtubules that is a requisite for efficient spindle assembly. As a result of this dampening, microtubules are unable to grow and shrink rapidly, and their ability to bind to condensed chromosomes during mitosis is compromised. Efficient chromosome alignment is thus affected, and this failure of chromosome alignment leads to mitotic delays mediated via the spindle assembly checkpoint.

It has been well established that antimitotic compounds compromise the ability of cells to execute a successful division. Cells will either fail to divide with a prolonged mitotic arrest that leads directly to cell death, or they divide abnormally, with an unequal distribution of DNA. Following such an unsuccessful division, cells may continue to cycle or undergo cell-cycle arrest or death.

The toxicities associated with paclitaxel and docetaxel are similar, and include neutropenia as the major dose limiting toxicity, along with significant peripheral neuropathy. In fact, dose reductions are frequent in heavily pretreated patients to mitigate the severity of these toxicities.

Protein kinases also play a critical role in the cell reproduction process. Specifically, the mitogen activated protein kinase (MAPK) signalling pathways consists of a kinase cascade that relays extracellular signals to the nucleus to regulate gene expression and key cellular functions. Gene expression controlled by the Ras/Raf/MEK/ERK signaling pathway regulates fundamental cellular processes including proliferation, differentiation, apoptosis, and angiogenesis. These diverse roles of Ras/Raf/MEK/ERK signaling are aberrantly activated in various types of cancer. Mutations in genes within this pathway may lead to constitutively active proteins resulting in increased cell proliferation, and resistance to apoptosis.

Raf (a serine/threonine-protein kinase) is encoded by a gene family consisting of three genes affording three Raf isoform members (B-Raf, C-Raf (Raf-1) and A-Raf). Each of these proteins share highly conserved amino-terminal regulatory regions and catalytic domains at the carboxy terminus. Although each isoform plays a role in the Ras/Raf/MEK/ERK pathway, B-Raf has been shown to be the main activator of MEK. B-Raf is recruited by Ras:GTP to the intracellular cell membrane where B-Raf becomes activated. In turn, B-Raf is responsible for activation of MEK1/2 and MEK1/2 activate ERK1/ERK2. Mutations in the B-Raf gene allow for B-Raf to signal independently of upstream signals. As a result, mutated B-Raf protein (such as V600E) causes excessive downstream signaling of MEK and ERK. This leads to excessive cell proliferation and survival and oncogenesis. Overactivation of the signaling cascade by mutated B-Raf has been implicated in multiple malignancies. B-Raf specific inhibitors (such as vemurafenib) are in fact, showing promise for the treatment of melanomas that express mutant B-Raf V600E, however the emergence of resistant disease is a growing concern. "A Phase 1B Study of MLN2480 in Combination With MLN0128 or Alisertib, or Paclitaxel, or Cetuximab, or Irinotecan in Adult Participants With Advanced Nonhematologic Malignancies" (20141230), ClinicalTrials.gov (URL: https://clinicaltrials.gov/ct2/show/NCT02327169) describes a study, with the primary purpose of determining the safety profile and the maximum tolerated doses (MTDs)/ potential recommended phase 2 doses (RP2Ds) of

the combination treatments of MLN2480 + MLN0128, MLN2480 + alisertib, MLN2480 + paclitaxel, MLN2480 + cetuximab, and MLN2480 + irinotecan in participants with advanced nonhematologic malignancies. Balani et al (Eur. J. Cancer, 50, suppl. 6, 2014, 54-55) describes drug-drug interaction predictions for MLN2480 based on non-clinical data. WO 2009/006389 describes compounds useful as inhibitors of Raf protein kinase, compositions thereof, and methods of treating Raf-mediated diseases. WO 2010/064722 describes a fused heterocyclic derivative showing Raf inhibitory activity.

It would be beneficial if more effective treatment regimens could be developed. Combinations with a Raf inhibitor that inhibits more isoforms of Raf proteins than B-Raf V600E mutation could be helpful for the treatment of cancer, and might potentially even overcome the resistance to a particular anticancer agent. Given the importance of the protein kinases involved in signal transduction pathways and the cell division cycle, combinations of a Raf inhibitor with a taxane might be particularly effective. Combinations of a Raf inhibitor with a taxane may have additive, or even synergistic, therapeutic effects. Thus, there is a need for new cancer treatment regimens, including combination therapies.

## SUMMARY OF THE INVENTION

The present disclosure relates to methods of treating a subject suffering from cancer, comprising administering to the subject:

(i) a Raf kinase inhibitor or a pharmaceutically acceptable salt thereof; and
(ii) a taxane or a pharmaceutically acceptable salt thereof; the amount of the Raf kinase inhibitor and taxane or a pharmaceutically acceptable salt thereof is such that the combination thereof is therapeutically effective in the treatment of the cancer.

In some embodiments, the Raf kinase inhibitor inhibits more than the B-Raf V600 isoform of Raf proteins. The Raf inhibitor is Compound A. The taxane is docetaxel, or a pharmaceutically acceptable salt thereof. In some embodiments, the cancer is a K-Ras exon 2 mutation positive cancer. In some embodiments, the cancer is a B-Raf mutation positive cancer. In some embodiments, the cancer is a non-V600 B-Raf mutation positive cancer. In some embodiments, the cancer is skin cancer, ocular cancer, gastrointestinal cancer, thyroid cancer, breast cancer, ovarian cancer, central nervous system cancer, laryngeal cancer, cervical cancer, lymphatic system cancer, genitourinary tract cancer, bone cancer, biliary tract cancer, endometrial cancer, uterine cancer, liver cancer, lung cancer, prostate cancer, or colon cancer.

The present invention relates to methods of treating a subject suffering from cancer, comprising administering to the subject:

(i) Compound A or a pharmaceutically acceptable salt thereof; and
(ii) docetaxel or a pharmaceutically acceptable salt thereof; the amount of said Compound A and docetaxel or a pharmaceutically acceptable salt thereof is such that the combination thereof is therapeutically effective in the treatment of the cancer.

In some embodiments, the cancer is non-small cell lung cancer. In some embodiments, the non-small cell lung cancer is a K-Ras mutation positive cancer. In some embodiments, the cancer is a K-Ras exon 2 mutation positive cancer. In some embodiments, the non-small cell lung cancer is a B-Raf mutation positive cancer. In some embodiments, the cancer is a non-V600 B-Raf mutation positive cancer. In some embodiments, Compound A or a pharmaceutically acceptable salt thereof, is administered administered once weekly (QW) with a rest period of 6 days between each administration in an amount of up to 600 mg per dose. In some embodiments, Compound A or a pharmaceutically acceptable salt thereof, is administered once weekly in an amount of about 400 mg to about 600 mg per dose and the taxane is paclitaxel or a pharmaceutically acceptable salt thereof administered in an amount of from about 70 mg/m$^2$ to about 90 mg/m$^2$ per dose.

The present disclosure relates to a pharmaceutical composition comprising:

(i) a Raf kinase inhibitor or a pharmaceutically acceptable salt thereof; and
(ii) a taxane or a pharmaceutically acceptable salt thereof; the amount of said Raf kinase inhibitor and taxane or a pharmaceutically acceptable salt thereof is such that the combination thereof is effective for extending the duration of response.

The Raf kinase inhibitor is Compound A.

## BRIEF DESCRIPTION OF THE DRAWINGS

Figure 1 is a graph that shows the mean tumor volume over time in female athymic NCr-*nu*/*nu* mice bearing SK-MEL-2 human melanoma xenografts dosed with vehicle, Compound A (12.5 mg/kg, QD) or docetaxel as single agents or in combination.

Figure 2 a graph that shows the mean tumor volume over time in female athymic NCr-*nu*/*nu* mice bearing SK-MEL-2 human melanoma xenografts dosed with vehicle, Compound A (50 mg/kg, BIW) or docetaxel as single agents or in combination.

Figure 3 is a graph that shows the average tumor volume over time in a Calu-6 lung cancer model (KRAS mutant $Q^{61K}$) dosed with Compound A (12.5 mg/kg, QD) or taxotere as single agents or in combination.

Figure 4 is a graph that shows average tumor volume over timer in a Calu-6 lung cancer model (KRAS mutant $Q^{61K}$) dosed with Compound A (50 mg/kg, Q3D) or taxotere as single agents or in combination.

## DESCRIPTION OF THE DISCLOSURE

The present disclosure provides new combination therapies for the treatment of cancers. In particular, the present disclosure provides a method of treating a subject suffering from cancer, comprising administering to the subject: (i) a first composition comprising, as an active agent, a Raf inhibitor or a pharmaceutically acceptable salt thereof; and (ii) a second composition comprising, as an active agent, a taxane or a pharmaceutically acceptable salt thereof; the amount of said active agents being such that the combination thereof is therapeutically effective in the treatment of cancer.

Terms used herein shall be accorded the following defined meanings, unless otherwise indicated.

As used herein, the term "Raf kinase" refers to any one of a family of serine/threonine-protein kinases. The family consists of three isoform members (B-Raf, C-Raf (Raf-1), and A-Raf). Raf protein kinases are involved in the MAPK signaling pathway consisting of a kinase cascade that relays extracellular signals to the nucleus to regulate gene expression and key cellular functions. Unless otherwise indicated by context, the term "Raf kinase" is meant to refer to any Raf kinase protein from any species, including, without limitation. In one aspect, the Raf kinase is a human Raf kinase.

The term "Raf inhibitor" or "inhibitor of Raf' is used to signify a compound which is capable of interacting with one or more isoform members (B-Raf, C-Raf (Raf-1) and/or A-Raf) of the serine/threonine-protein kinase, Raf including mutant forms. Some examples of Raf mutant forms include, but are not limited to B-Raf V600E, B-Raf V600D, B-Raf V600K, B-Raf V600E + T5291 and/or B-Raf V600E + G468A.

In some embodiments, the Raf kinase is at least about 50% inhibited, at least about 75% inhibited, at least about 90% inhibited, at least about 95% inhibited, at least about 98% inhibited, or at least about 99% inhibited. In some embodiments, the concentration of Raf kinase inhibitor required to reduce Raf kinase activity by 50% is less than about 1 μM, less than about 500 nM, less than about 100 nM, less than about 50 nM, less than about 25 nM, less than about 10 nM, less than about 5 nM, or less than about 1 nM.

In some embodiments, such inhibition is selective for one or more Raf isoforms, i.e., the Raf inhibitor is selective for one or more of B-Raf (wild type), mutant B-Raf, A-Raf, and C-Raf kinase. In some embodiments, the Raf inhibitor is selective for B-Raf (wild type), B-Raf V600E, A-Raf and C-Raf. In some embodiments, the Raf inhibitor is selective for B-Raf (wild type), B-Raf V600E, A-Raf and C-Raf. In some embodiments, the Raf inhibitor is selective for B-Raf (wild type), B-Raf V600D, A-Raf and C-Raf. In some embodiments, the Raf inhibitor is selective for B-Raf (wild type), B-Raf V600K, and C-Raf. In some embodiments, the Raf inhibitor is selective for more than B-Raf V600. In some embodiments, the Raf inhibitor is selective for more than B-Raf V600E.

In some embodiments, the Raf inhibitor is selective for B-Raf and C-Raf kinases. In some embodiments, the Raf inhibitor is selective for B-Raf(wild type), B-Raf V600E and C-Raf. In some embodiments, the Raf inhibitor is selective for B-Raf (wild type), B-Raf V600D and C-Raf. In some embodiments, the Raf inhibitor is selective for B-Raf (wild type), B-Raf V600K and C-Raf. In some embodiments, the Raf inhibitor is selective for mutant B-Raf. In some embodiments, the Raf inhibitor is selective for mutant B-Raf V600E. In some embodiments, the Raf inhibitor is selective for mutant B-Raf V600D. In some embodiments, the Raf inhibitor is selective for mutant B-Raf V600K.

The term "pan-Raf inhibitor" is a Raf inhibitor that inhibits more than the B-Raf V600 isoform of Raf proteins.

As used herein, the term "taxane" refers to a class of diterpenes produced by the plants of the genus Taxus (yews). Examples of taxanes include, but are not limited to, paclitaxel (TAXOL®), docetaxel (TAXOTERE®), and ABRAXANE® (Paclitaxel Injection).

The term "about" is used herein to mean approximately, in the region of, roughly, or around. When the term "about" is used in conjunction with a numerical range, it modifies that range by extending the boundaries above and below the numerical values set forth. In general, the term "about" is used herein to modify a numerical value above and below the stated value by a variance of 10%.

As used herein, the term "comprises" means "includes, but is not limited to."

As used herein, the terms "treatment," "treat," and "treating" are meant to include the full spectrum of intervention for the cancer from which the subject is suffering, such as administration of the combination to alleviate, slow, stop, or reverse one or more symptoms of the cancer and to delay the progression of the cancer even if the cancer is not actually eliminated. Treatment can include, for example, a decrease in the severity of a symptom, the number of symptoms, or frequency of relapse, e.g., the inhibition of tumor growth, the arrest of tumor growth, or the regression of already existing tumors.

The term "therapeutically effective amount" as used herein to refer to combination therapy means the amount of the combination of agents taken together so that the combined effect elicits the desired biological or medicinal response, i.e., either destroys the target cancer cells or slows or arrests the progression of the cancer in a subject. For example, the "therapeutically effective amount" as used herein to refer to combination therapy would be the amount of the Raf inhibitor and the amount of the taxane that when administered together, either sequentially or simultaneously, on the same or different days during a treatment cycle, has a combined effect that is beneficial. In some embodiments, the combined effect is additive. In some embodiments, the combined effect is synergistic. Further, it will be recognized by one skilled in the art that in the case of combination therapy with a therapeutically effective amount, as in the example above, the amount of the Raf inhibitor and/or the amount of the taxane individually may or may not be therapeutically effective.

"Cytotoxic effect," in reference to the effect of an agent on a cell, means killing of the cell. "Cytostatic effect" means an inhibition of cell proliferation. A "cytotoxic agent" means an agent that has a cytotoxic or cytostatic effect on a cell, thereby depleting or inhibiting the growth of, respectively, cells within a cell population.

In some embodiments, the growth of cells contacted with a Raf inhibitor and a taxane is inhibited by at least about 50% as compared to growth of non-contacted cells. In some embodiments, cell proliferation of contacted cells is inhibited by at least about 75%, at least about 90%, or at least about 95% as compared to non-contacted cells. In some embodiments, the phrase "inhibiting cell proliferation" includes a reduction in the number of contacted cells, as compare to non-contacted cells. Thus, a Raf inhibitor and a taxane that inhibits cell proliferation in a contacted cell may induce the contacted cell to undergo growth retardation, to undergo growth arrest, to undergo programmed cell death (i.e., apoptosis), or to undergo necrotic cell death.

The term "subject", as used herein, means a mammal, and "mammal" includes, but is not limited to a human. In some embodiments, the subject has been treated with an agent, e.g., a Raf inhibitor or a taxane, prior to initiation of treatment according to the method of the disclosure. In some embodiments, the subject is at risk of developing or experiencing a recurrence of a cancer.

Unless otherwise stated, structures depicted herein are meant to include compounds which differ only in the presence of one or more isotopically enriched atoms. For example, compounds having the present structure except for the replacement of a hydrogen atom by a deuterium or tritium, or the replacement of a carbon atom by a $^{13}C$- or $^{14}C$-enriched carbon are within the scope of the disclosure.

It will be apparent to one skilled in the art that certain compounds described herein may exist in tautomeric forms, all such tautomeric forms of the compounds being within the scope of the disclosure. Unless otherwise stated, structures depicted herein are also meant to include all stereochemical forms of the structure; i.e., the R and S configurations for each asymmetric center. Therefore, single stereochemical isomers as well as enantiomeric and diastereomeric mixtures of the present compounds are within the scope of the disclosure.

Compounds capable of inhibiting the activity of a Raf kinase maybe be used in the methods of the instant disclosure. In some embodiments, the Raf inhibitor inhibits more isoforms of Raf kinase proteins than B-Raf V600. In some embodiment, the Raf inhibitor inhibits more isoforms of RAf kinase proteins than B-Raf V600E. In some embodiments, the Raf inhibitor inhibits B-Raf (wild-type), mutant B-Raf, A-Raf, and C-Raf. In some embodiments, the Raf inhibitor is selective for B-Raf (wild-type), B-Raf V600E, A-Raf and C-Raf. In some embodiments, the Raf inhibitor is selective for B-Raf (wild-type), B-Raf V600E, A-Raf and C-Raf. In some embodiments, the Raf inhibitor is selective for B-Raf (wild-type), B-Raf V600D, A-Raf and C-Raf. In some embodiments, the Raf inhibitor is selective for B-Raf (wild-type), B-Raf V600K, and C-Raf. In some embodiments, the Raf inhibitor is selective for B-Raf (wild-type), B-Raf V600E and C-Raf. In some embodiments, the Raf inhibitor is selective for B-Raf (wild-type), B-Raf V600D and C-Raf. In some embodiments, the Raf inhibitor is selective for B-Raf (wild-type), B-Raf V600K and C-Raf. In some embodiments, the Raf inhibitor is selective for mutant B-Raf. In some embodiments, the Raf inhibitor is selective for mutant B-Raf V600E. In some embodiments, the Raf inhibitor is selective for mutant B-Raf V600D. In some embodiments, the Raf inhibitor is selective for mutant B-Raf V600K.

In particular, Raf inhibitors include the compounds described herein, as well as compounds disclosed in, for example, WO 2006/065703, WO 2010/064722, WO 2011/117381, WO 2011/090738, WO 2011/161216, WO 2011/097526, WO 2011/025927, WO 2011/023773, WO 2011/147764, WO 2011/079133, and WO 2011/063159. B-Raf specific inhibitors include vemurafinib (Roche), dabrafenib (GSK), and encoratinib (Novatis). Also suitable for use in the methods of the disclosure are solvated and hydrated forms of any of these compounds. Also suitable for use in

the methods of the disclosure are pharmaceutically acceptable salts of any of the compounds, and solvated and hydrated forms of such salts. These Raf inhibitors can be prepared in a number of ways well known to one skilled in the art of organic synthesis, including, but not limited to, the methods of synthesis described in detail in the above references.

In some embodiments, the Raf inhibitor is a compound that inhibits more isoforms of Raf kinase proteins than B-Raf V600. In particular, inhibitors of Raf kinase that inhibit more isoforms of Raf kinase proteins than B-Raf V600 include for example, compounds disclosed in WO 2009/006389, and US 2013/0252977 (DP-4978/ LY3009120), and including but not limited to compounds RAF-265, ARQ-736, CEP-32496, CCT 196969, CCT 241161, and REDX-04988

Raf inhibitors can be assayed in vitro or in vivo for their ability to bind to and/or inhibit Raf kinases. In vitro assays include biochemical FRET assays to measure the phophorylation of MEK by Raf kinases as a method for quantifying the ability of compounds to inhibit the enzymatic activity of Raf kinases. The compounds also can be assayed for their ability to affect cellular or physiological functions mediated by Raf kinase activity. For example in vitro assays quantitate the amount of phosphor-ERK in cancer cells. Assays for each of these activities are known in the art.

According to the present invention, the Raf inhibitor is (R)-2-(1-(6-amino-5-chloropyrimidine-4-carboxamide)ethyl)-N-(5-chloro-4-(trifluoromethyl)pyridin-2-yl)thiazole-5 -carboxamide (Compound A) or a pharmaceutically acceptable salt thereof:

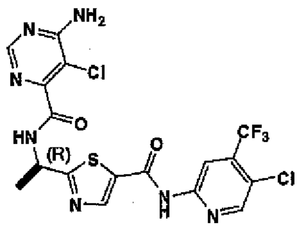

(Compound A).

Compound A is described in WO 2009/006389.

Also disclosed is the Raf inhibitor *N*-{7-cyano-6-[4-fluoro-3-({[3-(trifluoromethyl)-phenyl]acetyl}amino)phenoxy]-1,3-benzothiazol-2-yl}cyclopropanecarboxamide (Compound B) or a pharmaceutically acceptable salt thereof;

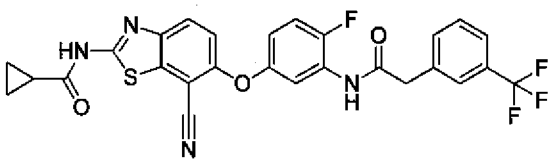

(Compound B).

Compound B is described in WO 2010/064722. Raf inhibitors, such as Compound A and Compound B that can inhibit more isoforms of Raf kinase proteins that B-Raf V600 have the ability to inhibit both Raf monomer and dimer-mediated signaling, which is a key feature that distinguishes these Raf inhibitors from recently approved B-Raf specific inhibitors (vemurafenib and dabrafenib).

As used herein, the term "pharmaceutically acceptable salt" refers to those salts which are, within the scope of sound medical judgment, suitable for use in contact with the tissues of humans and lower animals without undue toxicity, irritation, allergic response and the like, and are commensurate with a reasonable benefit/risk ratio. A "pharmaceutically acceptable salt" means any non-toxic salt of a compound disclosed herein that, upon administration to a recipient, is capable of providing, either directly or indirectly, the compound or an active metabolite or residue thereof.

Pharmaceutically acceptable salts are well known in the art. For example, S. M. Berge et al., describe pharmaceutically acceptable salts in detail in J. Pharmaceutical Sciences, 1977, 66, 1-19, Pharmaceutically acceptable salts of compounds described herein include those derived from suitable inorganic and organic acids and bases. Examples of pharmaceutically acceptable, nontoxic acid addition salts are salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid or malonic acid or by using other methods used in the art such as ion exchange. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate,

stearate, succinate, sulfate, tartrate, thiocyanate, p-toluenesulfonate, undecanoate, valerate salts, and the like. Salts derived from appropriate bases include alkali metal, alkaline earth metal, ammonium and $N^+(C_{1-4}alkyl)_4$ salts. The present disclosure also envisions the quaternization of any basic nitrogen-containing groups. Water or oil-soluble or dispersable products may be obtained by such quaternization. Representative alkali or alkaline earth metal salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, loweralkyl sulfonate and aryl sulfonate.

Compounds that are in the class of diterpenes produced by the plants of the genus Taxus (yews) may be used by the methods described in the instant disclosure. In particular, taxanes include but are not limited to the compounds described herein, paclitaxel (TAXOL®), docetaxel (TAXOTERE®), and ABRAXANE® (protein-bound paclitaxel for injection). In some embodiments, the taxane is paclitaxel or docetaxel or a pharmaceutically acceptable salt of paclitaxel or docetaxel. In some embodiments, the taxane is paclitaxel or a pharmaceutically acceptable salt thereof. In some embodiments, the taxane is docetaxel or a pharmaceutically acceptable salt thereof. In some embodiments, the taxane is paclitaxel. According to the present invention, the taxane is docetaxel.

In another aspect, the disclosure provides a pharmaceutical composition comprising i) a Raf inhibitor and ii) a taxane. The present disclosure provides new combination therapies for the treatment of cancers. In particular, the present disclosure provides a method of treating a subject suffering from cancer, comprising administering to the subject: (i) a first composition comprising, as an active agent, a Raf inhibitor or a pharmaceutically acceptable salt there; and (ii) a second composition comprising, as an active agent, a taxane or a pharmaceutically acceptable salt thereof; the amount of said active agents being such that the combination thereof is therapeutically effective in the treatment of cancer. The Raf inhibitor is Compound A and the taxane is docetaxel.

In some embodiments, the cancer is a solid tumor. In some embodiments, the cancer is a hematological malignancy. In some embodiments, the cancer is relapsed. In one aspect, relapsed cancer is cancer which has returned after a period of time in which no cancer could be detected.

In some embodiments, the cancer is refractory. In one aspect, refractory cancer does not respond to cancer treatment; it is also known as resistant cancer. In some embodiments, the tumor is unresectable. In one aspect, an unresectable tumor is unable to be removed by surgery. In some embodiments, the cancer has not been previously treated. In some embodiments, the cancer is locally advanced. In one aspect, "locally advanced" refers to cancer that is somewhat extensive but still confined to one area. In some instances, "locally advanced" can refer to a small tumor that hasn't spread but has invaded nearby organs or tissues that make it difficult to remove with surgery alone. In some embodiments, the cancer is metastatic. In one aspect, metastatic cancer is a cancer that has spread from the part of the body where it started (the primary site) to other parts of the body.

In some embodiments, the cancer is B-Raf, N-Ras, and/or K-Ras postitive cancer.

As used herein, "BRAF" or "B-Raf" refers to B-Raf proto-oncogene, serine/threonine kinase, the gene associated with the mRNA sequence assigned as GenBank Accession No. NM 004333, SEQ ID NO:1 (open reading frame is SEQ ID NO:2, nucleotides 62 to 2362 of SEQ ID NO: 1), encoding GenPept Accession No. NP_004324, SEQ ID NO:3). Other names for B-Raf include rafB1 and Noonan Syndrome 7 (NS7). B-Raf functions as a serine/threonine kinase, has a role in regulating the MAP kinase/ERKs signaling pathway and can be found on chromosome 7q.

As used herein, "KRAS" or "K-Ras" refers to v-Ki-ras2 Kirsten rat sarcoma viral oncogene homolog, the gene associated with the mRNA sequence assigned as GenBank Accession No. NM_004985, SEQ ID NO:4 (open reading frame is SEQ ID NO:5, nucleotides 193 to 759 of SEQ ID NO:4), encoding GenPept Accession No. NP_004976, SEQ ID NO:6, the predominant transcript variant of K-Ras gene on chromosome 12. Other names for K-Ras include KRAS2, and Noonan Syndrome 3 (NS3). K-Ras functions as an oncogene with GTPase activity and can be found on chromosome 12. K-Ras interacts with the cell membrane and various effector proteins, such as Akt and Cdc42, which carry out its signaling function through the cytoskeleton and effects on cell motility (Fotiadou et al. (2007) Mol. Cel. Biol. 27:6742-6755).

As used herein, "NRAS" or "N-Ras" refers to neuroblastoma RAS viral (v-ras) oncogene homolog, the gene associated with the mRNA sequence assigned as GenBank Accession No. NM_002524, SEQ ID NO:7 (open reading frame is SEQ ID NO:8, nucleotides 255 to 824 of SEQ ID NO:7), encoding GenPept Accession No. NP_002515, SEQ ID NO:9). Other names for N-Ras include Autoimmune Lymphoproliferative Syndrome type IV (ALPS4), NRAS1, and Noonan Syndrome 6 (NS6). N-Ras functions as an oncogene with GTPase activity and can be found on chromosome 1p. N-Ras interacts with the cell membrane and various effector proteins, such as Raf and RhoA, which carry out its signaling function through the cytoskeleton and effects on cell adhesion (Fotiadou et al. (2007) Mol. Cel. Biol. 27:6742-6755).

The combination in the disclosure may be used to treat a high unmet medical need cancer. In some embodiments, the combination is used to treat a genetically defined subset of cancer. In some embodiments, the cancer is B-Raf mutation-positive cancer (i.e., the cancer has one or more B-Raf mutations). In some embodiments, the B-Raf mutation is in exon 11 or 15. In some embodiments, the B-Raf mutation is in codon 466, 469, 594, 600, or 601. In some embodiments, the B-Raf mutation is in codon 600. In some embodiments, the B-Raf mutation includes but is not limited to a V600E,

V600D or V600K mutation. In some embodiments, the B-Raf mutation is V600E. In some embodiments, the B-Raf mutation is V600D. In some embodiments, the B-Raf mutation is V600K. In some embodiments, the B-Raf mutation is V600E + T5291. In some embodiments, the B-Raf mutation is V600E + G468A. "V600E mutation" means substitution of glutamic acid for valine at the amino acid position of 600. T529I is a threonine to isoleucine B-Raf gatekeeper mutation and G468A is a B-Raf secondary mutation at G1403C in exon 11. "V600K mutation" means substitution of lysine for valine at the amino acid position of 600. "V600D mutation" means substitution of aspartic acid for valine at the amino acid position of 600. The V600K mutation results in an amino acid substitution at position 600 in B-Raf, from a valine (V) to a lysine (K) The V600K mutation results in an amino acid substitution at position 600 in B-Raf, from a valine (V) to a lysine (K)).

In some embodiments, the cancer is a non-V600 B-Raf mutation positive cancer (i.e., the cancer has one or more B-Raf mutations and the one or more mutations is not B-Raf V600). In some embodiments, the B-Raf mutation is in exon 11 or 15. In some embodiments, the B-Raf mutation is in codon 466, 469, 594, or 601. In one aspect, one or more non-V600E mutation is G466A, G466V, N581S, D594H, R146W, L613F, D565_splice, S394*, P367R, G469A, G469V, G469*, G466V, G464V, G397S, S113I, A762E, G469L, D594N, G596S, G596R, D594N, D594H, or G327_splice. In one aspect, one or more non-V600E mutations are G469R, R95T, A621_splice, V639I, Q609H, G464V, or G466V. The asterisk "*" means a stop codon.

In some embodiments, the cancer is K-Ras mutation-positive cancer (i.e., the cancer has one or more K-Ras mutations). In some embodiments, the K-Ras mutation is in exon 2. In some embodiments, the K-Ras mutation is in codon 12 or 13.

In some embodiments, the cancer is N-Ras mutation-positive cancer (i.e., the cancer has one or more N-Ras mutations). In some embodiments, the N-Ras mutation is in exon 2, 3, or 4. In some embodiments, the N-Ras mutation is in exon 2. In some embodiments, the N-Ras mutation is in exon 3. In some embodiments, the N-Ras mutation is in exon 4. In some embodiments, the N-Ras mutation is Q61R, Q61K, Q61L, Q61H, or Q61P. In some embodiments, the N-Ras mutation is Q61R mutation.

The present disclosure provides a method of treating a subject suffering from cancer. In some embodiments, the cancer is selected from skin cancer, ocular cancer, gastrointestinal cancer, thyroid cancer, breast cancer, ovarian cancer, central nervous system cancer, laryngeal cancer, cervical cancer, lymphatic system cancer, genitourinary tract cancer, bone cancer, biliary tract cancer, endometrial cancer, liver cancer, lung cancer, prostate cancer and colon cancer. In some embodiments, lung cancer includes non-small cell lung cancer (NSCLC) and small cell lung cancer (SCLC). In some embodiments, the cancer is not NSCLC. In some embodiments, the cancer is selected from skin cancer, ocular cancer, gastrointestinal cancer, thyroid cancer, breast cancer, ovarian cancer, brain cancer, laryngeal cancer, cervical cancer, lymphatic system cancer, genitourinary tract cancer, bone cancer, biliary tract cancer, endometrial cancer, uterine cancer, liver cancer, lung cancer, prostate cancer and colon cancer.

In some embodiments, the cancer is a hematological malignancy. In some embodiments, the hematological malignancy is selected from acute myelogenous leukemia (AML), chronic myelogenous leukemia (CML), chronic lymphoblastic leukemia (CLL), and myelodysplastic syndrome.

In some embodiments, the cancer is selected from thyroid cancer, ovarian cancer, melanoma, acute myelogenous leukemia (AML), and colon cancer. In some embodiments, the cancer is melanoma or colon cancer.

In some embodiments, the cancer is selected from skin cancer and gastrointestinal cancer.

In some embodiments, the cancer is skin cancer. In some embodiments, the skin cancer is melanoma. In some embodiments, the melanoma is B-Raf-mutated melanoma. In some embodimentns, the melanoma is N-Ras-mutated melanoma.

In some embodiments, the cancer is lung cancer. In some embodiments, the cancer is non-small cell lung cancer (NSCLC). In some embodiments, the cancer is squamous NSCLC. In some embodiments, the cancer is non-squamous NSCLC.

In some embodiments, the cancer is gastrointestinal cancer. As used herein, "gastrointestinal cancer" includes cancer of the esophagus, stomach (also known as gastric cancer), biliary system, pancreas, small intestine, large intestine, rectum and anus). In some embodiments, the gastrointestinal cancer is adenocarcinoma of the esophagus, adenocarcinoma of the gastroesophageal junction or adenocarcinoma of the stomach. In some embodiments, the gastrointestinal cancer is stomach cancer.

In some embodiments, the cancer is colon cancer. Colon cancer is also known as colorectal (CRC), bowel, or rectum cancer.

In some embodiments, the cancer is a central nervous system cancer. In some embodiments, the central nervous system cancer is brain cancer.

In some embodiments, thyroid cancer is thyroid carcinoma.

In some embodiments, genitourinary tract cancer is bladder cancer.

In some embodiments, the hematogical malignancy is selected from acute myelogenous leukemia (AML) and chronic lymphocytic leukemia (CLL).

The Raf inhibitor and taxane are administered in such a way that they provide a beneficial effect in the treatment of a cancer. Administration can be by any suitable means provided that the administration provides the desired therapeutic effect, i.e., additivity or synergism. In some embodiments, the Raf inhibitor and taxane are administered during the same cycle of therapy, e.g., during one cycle of therapy, both the Raf kinse inhibitor and taxane are administered to the subject.

In some embodiments, the Raf inhibitor and taxane are cyclically administered to a subject. Cycling therapy involves the administration of a first agent (e.g., a first prophylactic or therapeutic agent) for a period of time, followed by the administration of a second agent and/or third agent (e.g., a second and/or third prophylactic or therapeutic agent) for a period of time and repeating this sequential administration. Cycling therapy can reduce the development of resistance to one or more of the therapies, avoid or reduce the side effects of one of the therapies, and/or improve the efficacy of the treatment.

In some embodiments, the treatment period during which an agent is administered is then followed by a non-treatment period of particular time duration, during which the therapeutic agents are not administered to the subject. This non-treatment period can then be followed by a series of subsequent treatment and non-treatment periods of the same or different frequencies for the same or different lengths of time. In some embodiments, the treatment and non-treatment periods are alternated. It will be understood that the period of treatment in cycling therapy may continue until the subject has achieved a complete response or a partial response, at which point the treatment may be stopped. Alternatively, the period of treatment in cycling therapy may continue until the subject has achieved a complete response or a partial response, at which point the period of treatment may continue for a particular number of cycles. In some embodiments, the length of the period of treatment may be a particular number of cycles, regardless of subject response. In some other embodiments, the length of the period of treatment may continue until the subject relapses.

The amounts or suitable dosages of the Raf inhibitor depends upon a number of factors, including the nature of the severity of the condition to be treated, the particular inhibitor, the route of administration and the age, weight, general health, and response of the individual subject. In some embodiments, the suitable dose level is one that achieves inhibition of more isoforms of B-Raf kinase proteins than B-Raf V600. In some embodiments, the suitable dose level is one that achieves inhibition of B-Raf, C-Raf, A-Raf and/or B-RafV600E. In some embodiments, the suitable dose level is one that achieves inhibition of B-Raf, C-Raf, and/or B-RafV600E. In some embodiments, the suitable dose level is one that achieves a therapeutic response as measured by tumor regression, or other standard measures of disease progression, progression free survival or overall survival. In some embodiments, the suitable dose level is one that achieves this therapeutic response and also minimizes any side effects associated with the administration of the therapeutic agent.

Suitable daily dosages of inhibitors of Raf kinase can generally range, in single or divided or multiple doses, from about 10% to about 100% of the maximum tolerated dose as a single agent. In some embodiments, the suitable dosages are from about 15% to about 100% of the maximum tolerated dose as a single agent. In some some embodiments, the suitable dosages are from about 25% to about 90% of the maximum tolerated dose as a single agent. In some other embodiments, the suitable dosages are from about 30% to about 80% of the maximum tolerated dose as a single agent. In some other embodiments, the suitable dosages are from about 40% to about 75% of the maximum tolerated dose as a single agent. In some other embodiments, the suitable dosages are from about 45% to about 60% of the maximum tolerated dose as a single agent. In some embodiments, suitable dosages are about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, about 100%, about 105%, or about 110% of the maximum tolerated dose as a single agent.

It will be understood that a suitable dosage of a Raf inhibitor may be taken at any time of the day or night. In some embodiments, a suitable dosage of a selective inhibitor of Raf inhibitor is taken in the morning. In some other embodiments, a suitable dosage of a Raf inhibitor is taken in the evening. In some other embodiments, a suitable dosage of a Raf inhibitor is taken both in the morning and the evening. It will be understood that a suitable dosage of a Raf inhibitor may be taken with or without food. In some embodiments a suitable dosage of a Raf inhibitor is taken with a meal. In some embodiments a suitable dosage of a Raf inhibitor is taken while fasting.

The present invention provides a method of treating a subject suffering from cancer, comprising administering to the subject: (i) a first composition comprising, as an active agent, Compound A or a pharmaceutically acceptable salt thereof; and (ii) a second composition comprising, as an active agent, docetaxel or a pharmaceutically acceptable salt thereof; the amount of said active agents being such that the combination thereof is therapeutically effective in the treatment of cancer. In some embodiments, Compound A is administered every other day (QOD) in an amount of from about 100 mg to about 200 mg per dose. In some some embodiments, Compound A is administered QOD in an amount of about 100 mg per dose. In some embodiments, Compound A is administered QOD in an amount of about 200 mg per dose.

In some embodiments, Compound A is administered from up to about 200 mg per dose. In some embodiments, Compound A is administered from up to 200 mg per dose. Suitable QOD dosages of a Raf inhibitor e.g., Compound A can generally range, in single or divided or multiple doses, from up to about 200 mg per dose. In some embodiments,

Compound A is administered as a single dose. In some embodiments, Compound A is administered as a divided dose. In some embodiments, Compound A is administered in multiple doses. Other suitable dosages of Compound A can generally range, in single or divided or multiple doses, from about 50 mg to about 200 mg per dose. Other suitable dosages of Compound A can generally range, in single or divided or multiple doses, from about 75 mg to about 200 mg per dose. In some embodiments, the suitable dosages are from about 100 mg to about 200 mg per dose. In some other embodiments, the suitable dosages are from about 150 mg to about 200 mg twice daily. In some embodiments, suitable dosages are about 20 mg, about 25 mg, about 30 mg, about 35 mg, about 40 mg, about 45 mg, about 50 mg, about 55 mg, about 60 mg, about 65 mg, about 70 mg, about 75 mg, about 80 mg, about 85 mg, about 90 mg, about 95 mg, about 100 mg, about 105 mg, about 110 mg, about 115 mg, about 120 mg, about 125 mg, about 130 mg, about 135 mg, about 140 mg, about 145 mg, about 150 mg, about 155 mg, about 160 mg, about 165 mg, about 170 mg, about 175 mg, about 180 mg, about 185 mg, about 190 mg, about 195 mg, or about 200 mg per dose. In some embodiments, the suitable dosage of Compound A is from about 100 mg to about 200 mg per dose.

Compound A is a Raf kinase inhibitor with a long half life which can support once weekly dosing (QW). In some embodiments, Compound A is administered once weekly with a rest period of 6 days between each administration. Suitable weekly dosages of a Raf inhibitor e.g., Compound A can generally range, in single or divided or multiple doses, from up to about 1500 mg once weekly (QW). Compound A can generally range, in single or divided or multiple doses, from up to 1500 mg once weekly (QW). In some embodiments, Compound A is administered from up to about 1000 mg once weekly. In some embodiments, Compound A is administered from up to 1000 mg once weekly. Once weekly means with a rest period of 6 days between each administration. In some embodiments, Compound A is administered as a single dose. In some embodiments, Compound A is administered QW in an amount of up to 600 mg per dose. In some embodiments, Compound A is administered in an amount of up to 600 mg per dose on days 2, 9, 16, and 23 of a 28-day cycle). In some embodiments, Compound A is administered as a divided dose. In some embodiments, Compound A is administered as a divided dose on the same day. In some embodiments, Compound A is administered in multiple doses. Suitable weekly dosages include from up to about 1000 mg per dose once a week with a rest period of 6 days between each administration. In some embodiments, Compound A is administered from up to 1000 mg per dose once a week with a rest period of 6 days between each administration. Other suitable weekly dosages of Compound A can generally range, in single or divided or multiple doses from about 200 mg to about 1000 mg per dose once a week. Other suitable weekly dosages of Compound A can generally range, in single or divided or multiple doses, from about 400 mg to about 1000 mg. In some embodiment, the suitable weekly dosage is from about 400 mg to about 900 mg per dose once a week. In some embodiments, the suitable weekly dosage is from about 500 mg to about 900 mg per dose once a week. In some other embodiments, the suitable weekly dosage is from about 400 mg to about 600 mg per dose once a week. In some other embodiments, the suitable weekly dosage is from about 200 mg to about 500 mg per dose once a week. In some other embodiments, the suitable weekly dosage is from about 200 mg to about 300 mg per dose once a week. In some embodiments, suitable weekly dosages are about 200 mg, 300 mg, about 400 mg, about 500 mg, about 600 mg, about 700 mg, about 800 mg, or about 900 mg per dose once a week. In some embodiments, the QW dosing schedule differentiates the combination of Compound A and taxane based on superior safety from other available therapies. In some embodiments, the QW dosing schedule differentiates the combination of Compound A and taxane based on superior efficacy from other available therapies.

The dosage of the Raf inhibitor administered to a subject will also depend on frequency of administration. In some embodiments, Compound A is administered once weekly (QW) with a rest period of 6 days between each administration. In some embodiments, Compound A is administered daily. In some embodiments, Compound A is administered every other day. In some embodiments, Compound A is administered on a 28-day cycle in which Compound A is administered on days 1, 3, 5, 8, 10, 12, 15, 17, 19,22,24, and 26 of a 28-day cycle. In some embodiments, Compound A is administered on a 28-day cycle in which Compound A is administered on days 2, 9, 16, and 23 of a 28-day cycle.

It will be readily apparent to those skilled in the art that other Raf inhibitor doses or frequencies of administration that provide the desired therapeutic effect are suitable for use in the present disclosure.

The taxane can be administered by any method known to one skilled in the art. For example, the taxane can be administered in the form of a composition, in one embodiment a pharmaceutical composition of a taxane and a pharmaceutically acceptable carrier, such as those described herein. In some embodiments, the pharmaceutical composition is a liquid dosage form, which can be administered via an intravenous route, such as intravenous injection or intravenous infusion. In one embodiment, paclitaxel is administered via intravenous injection. In another embodiment ABRAXANE® is administered via intravenous injection. Such pharmaceutical compositions are described in U.S. Patent No. 6096331 and U.S. Patent No. 6506405.

The amounts or suitable dosages of the taxane depends upon a number of factors, including the nature of the severity of the condition to be treated, the particular inhibitor, the route of administration and the age, weight, general health, and response of the individual subject. In some embodiments, the suitable dose level is one that achieves an effective exposure as measured by standard measures of effective exposure in cancer patients. In some embodiments, the suitable dose level is one that achieves a therapeutic response as measured by tumor regression, or other standard

measures of disease progression, progression free survival or overall survival. In some embodiments, the suitable dose level is one that achieves this therapeutic response and also minimizes any side effects associated with the administration of the therapeutic agent.

Suitable dosages of taxanes can generally range, in single or divided or multiple doses, from about 10% to about 100% of the maximum tolerated dose as a single agent. In some embodiments, the suitable dosages are from about 15% to about 100% of the maximum tolerated dose as a single agent. In some some embodiments, the suitable dosages are from about 25% to about 90% of the maximum tolerated dose as a single agent. In some other embodiments, the suitable dosages are from about 30% to about 80% of the maximum tolerated dose as a single agent. In some other embodiments, the suitable dosages are from about 40% to about 75% of the maximum tolerated dose as a single agent. In some other embodiments, the suitable dosages are from about 45% to about 60% of the maximum tolerated dose as a single agent. In some embodiments, suitable dosages are about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, about 100%, about 105%, or about 110% of the maximum tolerated dose as a single agent.

It will be understood that a suitable dosage of a taxane may be taken at any time of the day or night. In some embodiments, a suitable dosage of a taxane is taken in the morning. In some other embodiments, a suitable dosage of a taxane is taken in the evening. In some other embodiments, a suitable dosage of a taxane is taken both in the morning and the evening. It will be understood that a suitable dosage of a taxane may be taken with or without food. In some embodiments a suitable dosage of a taxane is taken with a meal. In some embodiments a suitable dosage of a taxane is taken while fasting.

Suitable dosages of paclitaxel can generally range, in single or divided or multiple doses, from about 40 $mg/m^2$ to about 90 $mg/m^2$ per week. Other suitable weekly dosages of paclitaxel can generally range, in single or divided or multiple doses, from about 70 $mg/m^2$ to about 90 $mg/m^2$ per week. Other suitable weekly dosages of paclitaxel can generally range, in single or divided or multiple doses, from about 60 $mg/m^2$ to about 90 $mg/m^2$ per week, or from about 60 $mg/m^2$ to about 80 $mg/m^2$ per week. In other embodiments, suitable dosages are about 40 $mg/m^2$, about 45 $mg/m^2$, about 50 $mg/m^2$, about 55 $mg/m^2$, about 60 $mg/m^2$, about 65 $mg/m^2$, about 70 $mg/m^2$, about 75 $mg/m^2$, about 80 $mg/m^2$, about 85 $mg/m^2$, or about 90 $mg/m^2$ per week. In some embodiments, the dosage of paclitaxel is about 80 $mg/m^2$.

Additionally, it will be appreciated that the taxane can be administered as an about 3-hour, about 2-hour, about 1-hour, about 45-minute, or about 30-min IV infusion. In some embodiments, the taxane can be administered by a 1-hour IV infusion.

In some embodiments, dosage of paclitaxel is about 80 $mg/m^2$ by a 1-hour IV infusion. In some embodiments,

Suitable dosages of ABRAXANE® can generally range from about 100 $mg/m^2$ to about 260 $mg/m^2$. In some embodiments, ABRAXANE is administered about 100 $mg/m^2$ intravenously per dose. In some embodiments, ABRAXANE is administered about 100 $mg/m^2$ intravenously over 30 min per dose on days 1, 8, and 15 of a 21-day cycle. In some embodiments, ABRAXANE is administered about 100 $mg/m^2$ intraveously over 30 min per dose on days 1, 8, and 15 of a 28-day cycle.

Suitable dosages of docetaxel can generally range from about 60 $mg/m^2$ to about 100 $mg/m^2$ intraveneously. In some embodiments, the dosage of docetaxel is administered about 75 $mg/m^2$ intraveneously. In some embodiments, dosage of docetaxel is administered about 75 $mg/m^2$ over 60 min every 3 weeks.

In some embodiments, a first treatment period in which a first amount of the taxane is administered can be followed by another treatment period in which a same or different amount of the same or a different taxane is administered. The second treatment period can be followed by other treatment periods. During the treatment and non-treatment periods, one or more additional therapeutic agents can be administered to the subject.

In some embodiments, the taxane is administered once weekly (QW) with a rest period of 6 days between each administration. In some embodiments, the taxane is administered on a 28-day cycle in which taxane is administered on days 1, 8, and 15.

Administration of the Raf inhibitor and the taxane can be on the same or different days provided that administration provides the desired thereapeutic effect. In some embodiments of the present disclosure, administration of the Raf inhibitor and the taxane will be on the same days. In some embodiments, administration of the Raf inhibitor and the taxane will be on the same and/or different days, e.g, Compound A is administered on days 1, 3, 5, 8, 10, 12, 15, 17, 19, 22, 24, and 26 of a 28-day cycle and the taxane is administered on days 1, 8, and 15 of a 28-day cycle. In some embodiments, Compound A is administered on days 2, 9, 16, and 23 of a 28-day cycle and the taxane is administered on days 1, 8, and 15 of a 28-day cycle. Alternative treatment schedules are encompassed by the present disclosure as long as they produce the desired result.

The taxane may be administered with the Raf inhibitor in a single dosage form or as a separate dosage form. When administered as a separate dosage form, the Raf inhibitor may be administered prior to, at the same time as, or following administration of the taxane.

In some embodiments, administration of a beneficial amount of the therapeutic agents encompasses administering Compound A or a pharmaceutically acceptable salt in an amount of from about 100 mg to about 200 mg per dose (measured amount of free Compound A) given on days 1, 3, 5, 8, 10, 12, 15, 17, 19, 22, 24, and 26 during the treatment cycle of 28-days in combination with administering paclitaxel or a pharmaceutically acceptable salt thereof in an amount of from about 70 mg/$m^2$ to about 90 mg/$m^2$ per dose (measured as the amount of free taxane) given on days 1, 8, and 15 during the treatment cycle of 28-days. In some embodiments, a beneficial amount of the therapeutic agents provides a synergistic benefit. In some embodiments, a beneficial amount of the therapeutic agents provides an additive benefit.

In some embodiments, the method to treat a subject suffering from cancer comprises administering to said subject a therapeutically effective amount of a combination of an amount of Compound A or a pharmaceutically acceptable salt thereof and an amount of a taxane or a pharmaceutically acceptable salt thereof. These cancer subjects include but are not limited to melonma subjects with a B-Raf mutation, melanoma subjects who failed vemurafenib or other B-Raf inhibitors, melanoma patients with N-Ras mutation B-Raf wild type, colorectal cancer subjects with B-Raf V600E mutation B-Raf wild type, ovarian cancer subjects with B-Raf V600E mutation B-Raf wild type, lung cancer subjects with B-Raf V600E mutation B-Raf wild type, AML subjects with N-Ras mutation B-Raf wild type, liver cancer subjects with N-Ras mutation B-Raf wild type, thyroid cancer subjects with B-Raf V600E or N-Ras mutation B-Raf wild type, pancreatic cancer with B-Raf wild type, bilary tract cancer subjects with B-Raf wild type. Cancer subjects include non-small cell lung cancer subjects with K-Ras exon 2 mutation or B-Raf mutation non-V600 B-Raf. In some embodiments, the cancer subject has failed standard therapies.

The disclosure provides a method for extending duration of response to treatment in subject suffering from cancer comprising administering to the subject: (i) a first composition comprising, as an active agent, a Raf inhibitor or a pharmaceutically acceptable salt thereof; and (ii) a second composition comprising, as an active agent, a taxane or a pharmaceutically acceptable salt thereof; the amount of said active agents being such that the combination thereof is effective for extending the duration of response.

The Raf inhibitor can be administered by any method known to one skilled in the art. For example, the Raf inhibitor can be administered in the form of a first composition, in some embodiments as a pharmaceutical composition of a Raf inhibitor and a pharmaceutically acceptable carrier, such as those described herein. In some embodiments, the first composition is a solid dispersion extrudate as described in PCT application PCT/2015/022792 (WO 2015/148828), filed March 26, 2015. In some embodiments, the first composition is a solid dispersion extrudate comprising a vinylpyrrolidinone-vinyl acetate copolymer and one or more pharmaceutical acceptable excipients. In some embodiments, the copolymer is copovidone e.g, Kollidon® VA64. In some embodiments, the first composition is amorphous.

The taxane can be administered by any method known to one skilled in the art. For example, the taxane can be administered in the form of a second composition, in some embodiments a pharmaceutical composition of the taxane and a pharmaceutically acceptable carrier, such as those described herein.

If a pharmaceutically acceptable salt of the Raf inhibitor or taxane is utilized in these compositions, the salt preferably is derived from an inorganic or organic acid or base. For reviews of suitable salts, see, e.g., Berge et al, J. Pharm. Sci. 66:1-19 (1977) and Remington: The Science and Practice of Pharmacy, 20th Ed., ed. A. Gennaro, Lippincott Williams & Wilkins, 2000.

Nonlimiting examples of suitable acid addition salts include the following: acetate, adipate, alginate, aspartate, benzoate, benzene sulfonate, bisulfate, butyrate, citrate, camphorate, camphor sulfonate, cyclopentanepropionate, digluconate, dodecylsulfate, ethanesulfonate, fumarate, lucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nicotinate, oxalate, pamoate, pectinate, persulfate, 3-phenyl-propionate, picrate, pivalate, propionate, succinate, tartrate, thiocyanate, tosylate and undecanoate.

Suitable base addition salts include, without limitation, ammonium salts, alkali metal salts, such as sodium and potassium salts, alkaline earth metal salts, such as calcium and magnesium salts, salts with organic bases, such as dicyclohexylamine, N-methyl-D-glucamine, t-butylamine, ethylene diamine, ethanolamine, and choline, and salts with amino acids such as arginine, lysine, and so forth.

Also, basic nitrogen-containing groups may be quaternized with such agents as lower alkyl halides, such as methyl, ethyl, propyl, and butyl chlorides, bromides and iodides; dialkyl sulfates, such as dimethyl, diethyl, dibutyl and diamyl sulfates, long chain halides such as decyl, lauryl, myristyl and stearyl chlorides, bromides and iodides, aralkyl halides, such as benzyl and phenethyl bromides and others. Water or oil-soluble or dispersible products are thereby obtained.

The term "pharmaceutically acceptable carrier" is used herein to refer to a material that is compatible with a recipient subject. In one aspect, the subject is a mammal. In one aspect, the subject is a human. In one aspect, the material is suitable for delivering an active agent to the target site without terminating the activity of the agent. The toxicity or adverse effects, if any, associated with the carrier preferably are commensurate with a reasonable risk/benefit ratio for the intended use of the active agent.

The terms "carrier", "adjuvant", or "vehicle" are used interchangeably herein, and include any and all solvents,

diluents, and other liquid vehicles, dispersion or suspension aids, surface active agents, isotonic agents, thickening or emulsifying agents, preservatives, solid binders, lubricants and the like, as suited to the particular dosage form desired. Remington: The Science and Practice of Pharmacy, 20th Ed., ed. A. Gennaro, Lippincott Williams & Wilkins, 2000 discloses various carriers used in formulating pharmaceutically acceptable compositions and known techniques for the preparation thereof. Except insofar as any conventional carrier medium is incompatible with the compounds of the disclosure, such as by producing any undesirable biological effect or otherwise interacting in a deleterious manner with any other components) of the pharmaceutically acceptable composition, its use is contemplated to be within the scope of this disclosure. Some examples of materials which can serve as pharmaceutically acceptable carriers include, but are not limited to, ion exchangers, alumina, aluminum stearate, lecithin, serum proteins, such as human serum albumin, buffer substances such as disodium hydrogen phosphate, potassium hydrogen phosphate, sodium carbonate, sodium bicarbonate, potassium carbonate, potassium bicarbonate, magnesium hydroxide and aluminum hydroxide, glycine, sorbic acid, or potassium sorbate, partial glyceride mixtures of saturated vegetable fatty acids, water, pyrogen-free water, salts or electrolytes such as protamine sulfate, disodium hydrogen phosphate, potassium hydrogen phosphate, sodium chloride, and zinc salts, colloidal silica, magnesium trisilicate, polyvinyl pyrrolidone, polyacrylates, waxes, polyethylene-polyoxypropylene-block polymers, wool fat, sugars such as lactose, glucose, sucrose, starches such as corn starch and potato starch, cellulose and its derivatives such as sodium carboxymethyl cellulose, ethyl cellulose and cellulose acetate, powdered tragacanth; malt, gelatin, talc, excipients such as cocoa butter and suppository waxes, oils such as peanut oil, cottonseed oil, safflower oil, sesame oil, olive oil, corn oil and soybean oil, glycols such as propylene glycol and polyethylene glycol, esters such as ethyl oleate and ethyl laurate, agar, alginic acid, isotonic saline, Ringer's solution, alcohols such as ethanol, isopropyl alcohol, hexadecyl alcohol, and glycerol, cyclodextrins, lubricants such as sodium lauryl sulfate and magnesium stearate, petroleum hydrocarbons such as mineral oil and petrolatum. Coloring agents, releasing agents, coating agents, sweetening, flavoring and perfuming agents, preservatives and antioxidants can also be present in the composition, according to the judgment of the formulator.

The pharmaceutical compositions of the disclosure can be manufactured by methods well known in the art such as conventional granulating, mixing, dissolving, encapsulating, lyophilizing, or emulsifying processes, among others. Compositions may be produced in various forms, including granules, precipitates, or particulates, powders, including freeze dried, rotary dried or spray dried powders, amorphous powders, tablets, capsules, syrup, suppositories, injections, emulsions, elixirs, suspensions or solutions. Formulations may optionally contain solvents, diluents, and other liquid vehicles, dispersion or suspension aids, surface active agents, pH modifiers, isotonic agents, thickening or emulsifying agents, stabilizers and preservatives, solid binders, lubricants and the like, as suited to the particular dosage form desired.

In some embodiments, the compositions of the disclosure are formulated for pharmaceutical administration to a mammal. In one aspect, for pharmaceutical administration to a human being. Such pharmaceutical compositions of the present disclosure may be administered orally, parenterally, by inhalation spray, topically, rectally, nasally, buccally, vaginally or via an implanted reservoir. The term "parenteral" as used herein includes subcutaneous, intravenous, intramuscular, intra-articular, intra-synovial, intrasternal, intrathecal, intrahepatic, intralesional and intracranial injection or infusion techniques. Preferably, the compositions are administered orally, intravenously, or subcutaneously. The formulations of the disclosure may be designed to be short-acting, fast-releasing, or long-acting. Still further, compounds can be administered in a local rather than systemic means, such as administration (e.g., by injection) at a tumor site.

Liquid dosage forms for oral administration include, but are not limited to, pharmaceutically acceptable emulsions, microemulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, cyclodextrins, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof. Besides inert diluents, the oral compositions can also include adjuvants such as wetting agents, emulsifying and suspending agents, sweetening, flavoring, and perfuming agents.

Injectable preparations, for example, sterile injectable aqueous or oleaginous suspensions may be formulated according to the known art using suitable dispersing or wetting agents and suspending agents. The sterile injectable preparation may also be a sterile injectable solution, suspension or emulsion in a nontoxic parenterally acceptable diluent or solvent, for example, as a solution in 1,3-butanediol. Among the acceptable vehicles and solvents that may be employed are water, Ringer's solution, U.S.P. and isotonic sodium chloride solution. In addition, sterile, fixed oils are conventionally employed as a solvent or suspending medium. For this purpose any bland fixed oil can be employed including synthetic mono- or diglycerides. In addition, fatty acids such as oleic acid are used in the preparation of injectables. The injectable formulations can be sterilized, for example, by filtration through a bacterial-retaining filter, or by incorporating sterilizing agents in the form of sterile solid compositions which can be dissolved or dispersed in sterile water or other sterile injectable medium prior to use. Compositions formulated for parenteral administration may be injected by bolus injection or by timed push, or may be administered by continuous infusion.

In order to prolong the effect of a compound of the present disclosure, it may be desirable to slow the absorption

of the compound from subcutaneous or intramuscular injection. This may be accomplished by the use of a liquid suspension of crystalline or amorphous material with poor water solubility. The rate of absorption of the compound then depends upon its rate of dissolution that, in turn, may depend upon crystal size and crystalline form. Alternatively, delayed absorption of a parenterally administered compound form is accomplished by dissolving or suspending the compound in an oil vehicle. Injectable depot forms are made by forming microencapsule matrices of the compound in biodegradable polymers such as polylactide-polyglycolide. Depending upon the ratio of compound to polymer and the nature of the particular polymer employed, the rate of compound release can be controlled. Examples of other biodegradable polymers include poly(orthoesters) and poly(anhydrides). Depot injectable formulations are also prepared by entrapping the compound in liposomes or microemulsions that are compatible with body tissues.

Compositions for rectal or vaginal administration are preferably suppositories which can be prepared by mixing the compounds of this disclosure with suitable non-irritating excipients or carriers such as cocoa butter, polyethylene glycol or a suppository wax which are solid at ambient temperature but liquid at body temperature and therefore melt in the rectum or vaginal cavity and release the active compound.

Solid dosage forms for oral administration include capsules, tablets, pills, powders, and granules. In such solid dosage forms, the active compound is mixed with at least one inert, pharmaceutically acceptable excipient or carrier such as sodium citrate or dicalcium phosphate and/or a) fillers or extenders such as starches, lactose, sucrose, glucose, mannitol, and silicic acid, b) binders such as, for example, carboxymethylcellulose, alginates, gelatin, polyvinylpyrrolidinone, sucrose, and acacia, c) humectants such as glycerol, d) disintegrating agents such as agar--agar, calcium carbonate, potato or tapioca starch, alginic acid, certain silicates, and sodium carbonate, e) solution retarding agents such as paRaffin, f) absorption accelerators such as quaternary ammonium compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. In the case of capsules, tablets and pills, the dosage form may also comprise buffering agents such as phosphates or carbonates.

Solid compositions of a similar type may also be employed as fillers in soft and hard-filled gelatin capsules using such excipients as lactose or milk sugar as well as high molecular weight polyethylene glycols and the like. The solid dosage forms of tablets, dragees, capsules, pills, and granules can be prepared with coatings and shells such as enteric coatings and other coatings well known in the pharmaceutical formulating art. They may optionally contain opacifying agents and can also be of a composition that they release the active ingredient(s) only, or preferentially, in a certain part of the intestinal tract, optionally, in a delayed manner. Examples of embedding compositions that can be used include polymeric substances and waxes. Solid compositions of a similar type may also be employed as fillers in soft and hard-filled gelatin capsules using such excipients as lactose or milk sugar as well as high molecular weight polyethylene glycols and the like.

The active compounds can also be in micro-encapsulated form with one or more excipients as noted above. The solid dosage forms of tablets, dragees, capsules, pills, and granules can be prepared with coatings and shells such as enteric coatings, release controlling coatings and other coatings well known in the pharmaceutical formulating art. In such solid dosage forms the active compound may be admixed with at least one inert diluent such as sucrose, lactose or starch. Such dosage forms may also comprise, as is normal practice, additional substances other than inert diluents, e.g., tableting lubricants and other tableting aids such a magnesium stearate and microcrystalline cellulose. In the case of capsules, tablets and pills, the dosage forms may also comprise buffering agents. They may optionally contain opacifying agents and can also be of a composition that they release the active ingredient(s) only, or preferentially, in a certain part of the intestinal tract, optionally, in a delayed manner. Examples of embedding compositions that can be used include polymeric substances and waxes.

Dosage forms for topical or transdermal administration of a compound of this disclosure include ointments, pastes, creams, lotions, gels, powders, solutions, sprays, inhalants or patches. The active component is admixed under sterile conditions with a pharmaceutically acceptable carrier and any needed preservatives or buffers as may be required. Ophthalmic formulation, ear drops, and eye drops are also contemplated as being within the scope of this disclosure. Additionally, the present disclosure contemplates the use of transdermal patches, which have the added advantage of providing controlled delivery of a compound to the body. Such dosage forms can be made by dissolving or dispensing the compound in the proper medium. Absorption enhancers can also be used to increase the flux of the compound across the skin. The rate can be controlled by either providing a rate controlling membrane or by dispersing the compound in a polymer matrix or gel.

Compositions for use in the method of the disclosure may be formulated in unit dosage form for ease of administration and uniformity of dosage. The expression "unit dosage form" as used herein refers to a physically discrete unit of agent appropriate for the subject to be treated. It will be understood, however, that the total daily usage of the compounds and compositions of the present disclosure will be decided by the attending physician within the scope of sound medical judgment. A unit dosage form for parenteral administration may be in ampoules or in multi-dose containers.

The disclosure includes a kit, comprising (i) a first composition comprising, as an active agent, a Raf inhibitor

or a pharmaceutically salt thereof; and (ii) a second composition comprising, as an active agent, taxane or a pharmaceutically acceptable salt thereof; and instructions for administering the first composition in combination with the second composition.

The disclosure includes a kit, comprising (i) a first composition comprising, as an active agent, a Raf inhibitor or a pharmaceutically salt thereof; and (ii) a second composition comprising, as an active agent, taxane or a pharmaceutically acceptable salt thereof when used to treat cancer in a subject; and instructions for administering the first composition in combination with the second composition.

Vemurafenib (Roche) was approved by the United States Food and Drug Administration (FDA) for treatment of melanoma patients with B-Raf V600E mutation. More recently, dabrafenib (B-Raf inhibitor) and trametinib (MEK inhibitor) were approved for patients with B-Raf V600E positive melanoma. Both drugs significantly improved the median progression-free survival compared with chemotherapy in Phase 3 studies. As is the case with vemurafinib, however, these responses are considered short-lived (Lancet (2012;380:358-365), N Engl J Med 2012; 367:107-114). Similar to many other targeted therapies, the acquired resistance to B-Raf inhibition presents a therapeutic challenge to long-term survival benefit in this patient population.

To improve the benefit of B-Raf inhibitors, research continues to identify the mechanisms which render mutant B-Raf expressing melanoma cells resistant to vemurafenib. Recent studies have indicated that reactivation of the MAPK pathway is a mechanism of resistance to B-Raf inhibition. Resistant mechanisms primarily involve reactivation of ERK signaling through bypass mechanisms that are either Ras/Raf dependent, such as N-Ras activation, Nazarian et al, Nature. 2010, 468: 973-7, H-Ras activation (Su et al, New England Journal of Medicine. 2012, 366: 207-215) or C-Raf upregulation, (Johannessen et al, Nature. 2010,468: 968-72; Montagut et al, Cancer Res. 2008, 68: 4853-61), aberrantly spliced variants of B-Raf V600E (Poulikakos et al, Nature. 2011, 480: 387-390, or Ras/Raf independent (Tpl2/COT overexpression) Johannessen et al, Nature. 2010, 468: 968-72. Consequently, multiple mechanisms could attenuate the effect of B-Raf inhibition on MAPK signaling in B-Raf mutant cancers. Although a gatekeeper mutation of B-Raf (T529I) that could cause resistance to B-Raf inhibition has not yet been clinically identified, such a mutation has been experimentally demonstrated to cause resistance, Whittaker et al, Sci Transl Med. 2010, 2(35): ra41. Recent studies have also suggested that activation of MAPK-redundant signaling pathways by RTKs such as IGF-1R or PDGFRβ could play a role in acquired resistance to B-Raf inhibition; Nazarian et al, Nature. 2010, 468: 973-7; Villanueva et al, Cancer Cell. 2010, 18: 683-95; Shi et al, Cancer Res. 2011, 71: 5067-74. It is clear that MAPK reactivation is involved in many of these resistance mechanisms. A pan Raf inhibitor is expected to block MAPK reactivation.

Additionally, B-Raf specific inhibitors including vemurafenib and its close analogue N-[3-(5-chloro-1H-pyrrolo[2,3-b]pyridine-3-carbonyl)-2,4-difluorophenyl]p-ropane-1-sulfonamide (PLX4720; a commercially available selective B-Raf inhibitor) were demonstrated to induce paradoxical pathway activation through dimerization with other Raf isoforms in a B-Raf wild type background, Hatzivassiliou G, et al. Nature, 2010, 464: 431-435; Poulikakos et al, Nature, 2010,464:427-430; Heidorn, et al, Cell, 2010, 140: 209-221. Vemurafenib is believed to activate the RafTMEK/ERK. pathway through binding B-Raf wild type and stimulating B-Raf-C-Raf dimerization. This paradoxical pathway activation by B-Raf specific inhibition is believed to be a major reason of skin side effects (such as squamous cell carcinoma) in some melanoma patients treated with vemurafenib. Vemurafenib is not approved for treatment of cancer patients with B-Raf wild type genetic background due to its paradoxical pathway activation activity in this genetic background.

Compound A is a Raf kinase inhibitor capable of inhibiting more isoforms of Raf kinase proteins than B-Raf V600. Specifically, Compound A inhibits B-Raf, C-Raf, and B-Raf V600E mutation (see Example 1). Due to its pan-Raf activities, Compound A is active against tumor cells with MAPK pathway activation by upstream signaling such as N-Ras mutation and K-Ras mutation, both with B-Raf wild type genetic background. Therefore, Compound A has the potential for treating cancer patients with B-Raf mutation (such as melanoma, colorectal, lung (NSCLC), ovarian and thyroid carcinoma) or N-Ras mutation, B-Raf wild type (such as melanoma, AML, CML, ALL, CLL, liver cancer), (Schubbert et al, Nature Reviews Cancer, 2007, 7: 295; Pylayeva-Gupta et al, Nature Reviews Cancer, 2011, 11: 761). Compound A is also active against melanoma tumor cells which developed resistance to vemurafenib. Therefore, it is believed that the Compound A, in combination with a taxane, will be effective for melanoma patients who have failed vemurafenib or other B-Raf inhibitors. In some embodiments, the combination of Compound A with paclitaxel is a better-tolerated combination that leads to the longest duration of therapy. The present disclosure relates to a method for extending duration of response to treatment in subject suffering from cancer comprising administering to the subject a pharmaceutical composition described herein.

The present disclosure relates to a method for determining whether to treat a subject suffering from cancer with a pharmaceutical composition decribed herein, said method comprising:

a) measuring at least one characteristic of at least one or more B-Raf, N-Ras and/or K-Ras markers associated with gene mutation in a subject sample comprising tumor cells;
b) identifying whether the at least one characteristic measured in step a) is informative for outcome upon treatment with the pharmaceutical composition; and

c) determining to treat the subject with the pharmaceutical composition if the informative characteristic indicates that the tumor cells comprise at least one marker gene with a B-Raf, N-Ras and/or K-Ras mutational status that indicates a favorable outcome to treatment with the pharmaceutical composition.

The present disclosure relates to a method of treating a subject suffering from cancer by administering to the subject a pharmaceutical composition decribed herein, said method comprising:

a) measuring at least one characteristic of at least one or more B-Raf, N-Ras and/or K-Ras markers associated with gene mutation in a subject sample comprising tumor cells;
b) identifying whether the at least one characteristic measured in step a) is informative for outcome upon treatment with the pharmaceutical composition; and
c) determining to treat the subject with the pharmaceutical composition if the informative characteristic indicates that the tumor cells comprise at least one marker gene with a B-Raf, N-Ras and/or K-Ras mutational status that indicates a favorable outcome to treatment with the pharmaceutical composition.

The present disclosure relates to a method for determining an increased likelihood of pharmacological effectiveness of treatment by a pharmaceutical composition of claim 16 or 17 in a subject diagnosed with cancer, said method comprising: subjecting a nucleic acid sample from a cancer (tumor) sample from the subject to B-Raf, N-Ras or K-Ras mutational testing or PCR, wherein the presence of at least one mutation in B-Raf, N-Ras or K-Ras gene indicates an increased likelihood of pharmacological effectiveness of the treatment.
The present disclosure relates to a method for treating a subject suffering from cancer by administering to a subject a pharmaceutical composition described herein, said method comprising: subjecting a nucleic acid sample from a cancer (tumor) sample from the subject to B-Raf, N-Ras or K-Ras mutational testing or PCR, wherein the presence of at least one mutation in B-Raf, N-Ras or K-Ras gene indicates an increased likelihood of pharmacological effectiveness of the treatment.
The present disclosure relates to a method of treating a subject having cancer, said method comprising:

i) obtaining a nucleic acid sample from a cancer sample from said subject;
ii) subjecting the sample to B-Raf, N-Ras or K-Ras mutational testing or PCR and identifying the presence of at least one mutation in B-Raf, N-Ras, or K-Ras gene; and administering an effective amount of a pharmaceutical composition described herein to the subject in whose sample the presence of at least one mutation in B-Raf, N-Ras or K-Ras gene is identified

In some embodiments, a mutation in a marker can be identified by sequencing a nucleic acid, e.g., a DNA, RNA, cDNA or a protein correlated with the marker gene, e.g., a genotype marker gene, e.g., B-Raf or N-Ras. There are several sequencing methods known in the art to sequence nucleic acids. A nucleic acid primer can be designed to bind to a region comprising a potential mutation site or can be designed to complement the mutated sequence rather than the wild type sequence. Primer pairs can be designed to bracket a region comprising a potential mutation in a marker gene. A primer or primer pair can be used for sequencing one or both strands of DNA corresponding to the marker gene. A primer can be used in conjunction with a probe, e.g., a nucleic acid probe, e.g., a hybridization probe, to amplify a region of interest prior to sequencing to boost sequence amounts for detection of a mutation in a marker gene. Examples of regions which can be sequenced include an entire gene, transcripts of the gene and a fragment of the gene or the transcript, e.g., one or more of exons or untranslated regions or a portion of a marker comprising a mutation site. Examples of mutations to target for primer selection and sequence or composition analysis can be found in public databases which collect mutation information, such as Database of Genotypes and Phenotypes (dbGaP) maintained by the National Center for Biotechnology Information (Bethesda, MD) and Catalogue of Somatic Mutations in Cancer (COSMIC) database maintained by the Wellcome Trust Sanger Institute (Cambridge, UK).
Sequencing methods are known to one skilled in the art. Examples of methods include the Sanger method, the SEQUENOM™ method and Next Generation Sequencing (NGS) methods. The Sanger method, comprising using electrophoresis, e.g., capillary electrophoresis to separate primer-elongated labeled DNA fragments, can be automated for high-throughput applications. The primer extension sequencing can be performed after PCR amplification of regions of interest. Software can assist with sequence base calling and with mutation identification. SEQUENOM™ MASSARRAY® sequencing analysis (San Diego, CA) is a mass-spectrometry method which compares actual mass to expected mass of particular fragments of interest to identify mutations. NGS technology (also called "massively parallel sequencing" and "second generation sequencing") in general provides for much higher throughput than previous methods and uses a variety of approaches (reviewed in Zhang et al. (2011) J. Genet. Genomics 38:95-109 and Shendure and Hanlee (2008) Nature Biotech. 26:1135-1145). NGS methods can identify low frequency mutations in a marker in a sample. Some NGS methods (see, e.g., GS-FLX Genome Sequencer (Roche Applied Science, Branford, CT), Genome analyzer

(Illumina, Inc. San Diego, CA) SOLID™ analyzer (Applied Biosystems, Carlsbad, CA), Polonator G.007 (Dover Systems, Salem, NH), HELISCOPE™ (Helicos Biosciences Corp., Cambridge, MA)) use cyclic array sequencing, with or without clonal amplification of PCR products spatially separated in a flow cell and various schemes to detect the labeled modified nucleotide that is incorporated by the sequencing enzyme (e.g., polymerase or ligase). In one NGS method, primer pairs can be used in PCR reactions to amplify regions of interest. Amplified regions can be ligated into a concatenated product. Clonal libraries are generated in the flow cell from the PCR or ligated products and further amplified ("bridge" or "cluster" PCR) for single-end sequencing as the polymerase adds a labeled, reversibly terminated base that is imaged in one of four channels, depending on the identity of the labeled base and then removed for the next cycle. Software can aid in the comparison to genomic sequences to identify mutations. Another NGS method is exome sequencing, which focuses on sequencing exons of all genes in the genome. As with other NGS methods, exons can be enriched by capture methods or amplification methods.

In some embodiments, DNA, e.g., genomic DNA corresponding to the wild type or mutated marker can be analyzed both by *in situ* and by *in vitro* formats in a biological sample using methods known in the art. DNA can be directly isolated from the sample or isolated after isolating another cellular component, e.g., RNA or protein. Kits are available for DNA isolation, e.g., QIAAMP® DNA Micro Kit (Qiagen, Valencia, CA). DNA also can be amplified using such kits.

In another embodiment, mRNA corresponding to the marker can be analyzed both by *in situ* and by *in vitro* formats in a biological sample using methods known in the art. Many expression detection methods use isolated RNA. For *in vitro* methods, any RNA isolation technique that does not select against the isolation of mRNA can be utilized for the purification of RNA from tumor cells (see, *e.g.,* Ausubel et al., ed., Current Protocols in Molecular Biology, John Wiley & Sons, New York 1987-1999). Additionally, large numbers of tissue samples can readily be processed using techniques well known to those of skill in the art, such as, for example, the single-step RNA isolation process of Chomczynski (1989, U.S. Patent No. 4,843,155). RNA can be isolated using standard procedures (see *e.g.,* Chomczynski and Sacchi (1987) Anal. Biochem. 162:156-159), solutions (*e.g.,* trizol, TRI REAGENT® (Molecular Research Center, Inc., Cincinnati, OH; see U.S. Patent No. 5,346,994) or kits (*e.g.,* a QIAGEN® Group RNEASY® isolation kit (Valencia, CA) or LEUKOLOCK™ Total RNA Isolation System, Ambion division of Applied Biosystems, Austin, TX).

Additional steps may be employed to remove DNA from RNA samples. Cell lysis can be accomplished with a nonionic detergent, followed by microcentrifugation to remove the nuclei and hence the bulk of the cellular DNA. DNA subsequently can be isolated from the nuclei for DNA analysis. In one embodiment, RNA is extracted from cells of the various types of interest using guanidinium thiocyanate lysis followed by CsCl centrifugation to separate the RNA from DNA (Chirgwin et al. (1979) Biochemistry 18:5294-99). Poly(A)+RNA is selected by selection with oligo-dT cellulose (see Sambrook et al. (1989) Molecular Cloning--A Laboratory Manual (2nd ed.), Cold Spring Harbor Laboratory, Cold Spring Harbor, N.Y.). Alternatively, separation of RNA from DNA can be accomplished by organic extraction, for example, with hot phenol or phenol/chloroform/isoamyl alcohol. If desired, RNAse inhibitors may be added to the lysis buffer. Likewise, for certain cell types, it may be desirable to add a protein denaturation/digestion step to the protocol. For many applications, it is desirable to enrich mRNA with respect to other cellular RNAs, such as transfer RNA (tRNA) and ribosomal RNA (rRNA). Most mRNAs contain a poly(A) tail at their 3' end. This allows them to be enriched by affinity chromatography, for example, using oligo(dT) or poly(U) coupled to a solid support, such as cellulose or SEPHADEX.R™. medium (see Ausubel et al. (1994) Current Protocols In Molecular Biology, vol. 2, Current Protocols Publishing, New York). Once bound, poly(A)+mRNA is eluted from the affinity column using 2 mM EDTA/0.1% SDS.

A characteristic of a marker in a sample, e.g., after obtaining a sample (*e.g.,* a a tumor biopsy) from a test subject, can be assessed by any of a wide variety of well known methods for detecting or measuring the characteristic, e.g., of a marker or plurality of markers, e.g., of a nucleic acid (*e.g.,* RNA, mRNA, genomic DNA, or cDNA) and/or translated protein. Non-limiting examples of such methods include immunological methods for detection of secreted, cell-surface, cytoplasmic, or nuclear proteins, protein purification methods, protein function or activity assays, nucleic acid hybridization methods, optionally including "mismatch cleavage" steps (Myers, et al. (1985) Science 230:1242) to digest mismatched, i.e. mutant or variant, regions and separation and identification of the mutant or variant from the resulting digested fragments, nucleic acid reverse transcription methods, and nucleic acid amplification methods and analysis of amplified products. These methods include gene array/chip technology, RT-PCR, TAQMAN® gene expression assays (Applied Biosystems, Foster City, CA), e.g., under GLP approved laboratory conditions, *in situ* hybridization, immunohistochemistry, immunoblotting, FISH (flourescence *in situ* hybridization), FACS analyses, northern blot, southern blot, INFINIUM® DNA analysis Bead Chips (Illumina, Inc., San Diego, CA), quantitative PCR, bacterial artificial chromosome arrays, single nucleotide polymorphism (SNP) arrays (Affymetrix, Santa Clara, CA) or cytogenetic analyses.

Examples of techniques for detecting differences of at least one nucleotide between two nucleic acids include, but are not limited to, selective oligonucleotide hybridization, selective amplification, or selective primer extension. For example, oligonucleotide probes can be prepared in which the known polymorphic nucleotide is placed centrally (allele- or mutant-specific probes) and then hybridized to target DNA under conditions which permit hybridization only if a perfect match is found (Saiki et al. (1986) Nature 324:163); Saiki et al (1989) Proc. Natl Acad. Sci USA 86:6230; and Wallace

et al. (1979) Nucl. Acids Res. 6:3543). Such allele specific oligonucleotide hybridization techniques can be used for the simultaneous detection of several nucleotide changes in different polymorphic or mutated regions of N-Ras. For example, oligonucleotides having nucleotide sequences of specific allelic variants or mutants are attached to a solid support, e.g., a hybridizing membrane and this support, e.g., membrane, is then hybridized with labeled sample nucleic acid. Analysis of the hybridization signal thus can reveal the identity of the nucleotides of the sample nucleic acid.

Unless defined otherwise, all technical and scientific terms used herein have the same meanings as commonly understood by one of ordinary skill in the art to which this disclosure belongs. Although any methods and materials similar or equivalent to those described herein can be used in the practice or testing of the present disclosure, the preferred methods, devices and materials are herein described.

## EXAMPLES

Definitions

| | |
|---|---|
| ANOVA | Analysis of variance |
| ΔAUC | difference in the area under the curve |
| BID | twice daily |
| BIW | twice weekly |
| BWL | body weight loss |
| HPBCD | 2-hydroxypropyl-β-cyclodextrin |
| HPMC | hydroxypropylmethylcellulose |
| IV | intravenous(ly) |
| MTD | maximum tolerated dose |
| N/A | not applicable |
| $NaHC0_3$ | Sodium bicarbonate |
| SEM | Standard error of the mean |
| SCID | severe combined immunodeficiency |
| PEG | Polyethylene glycol |
| po. | Orally (by mouth, *per os*) |
| QD | once daily |
| QW or Q7D | once weekly |
| SC | subcutaneous(ly) |
| TG | treatment group |
| TGI | tumor growth inhibition |
| WFI | water for injection |

### Example 1: Kinase inhibition assay with purified Raf kinase isoforms

The kinase activity of Compound A was determined using a biochemical fluorescence resonance energy transfer (FRET) assay as described in WO 2009/006389. The half maximal inhibitory concentration (IC50) values of Compound A for mutant B-Raf V600E, wild-type B-Raf, and wild-type C-Raf kinases is shown below in Table 1. Compound A binds to the inactive, DFG-out conformation of B-Raf kinase.

Table 1.

| Biochemical kinase assay | |
|---|---|
| Raf | $IC_{50}$ value (nM) |
| B-Raf mutant (V600E) | 7.1 |
| B-Raf wild-type | 10.1 |
| C-Raf wild-type | 0.7 |

**Example 2: In vivo tumor efficacy in N-Ras Mutated SK-MEL-2 human melanoma xenograft model**

Test Compounds:

Compound A was formulated in PEG 400 and the suspension was sonicated in a warm water bath until a clear solution was obtained. The 10 mg/mL solution was diluted with 100% PEG 400 for the lower dose.

Docetaxel [Taxotere® (docetaxel) Injection Concentrate; 20 mg/mL in Ethanol/Tween 80] was diluted with saline to 1.5 mg/mL. Only docetaxel was used in non-clinical studies because of formulation limitations in mice.

The 2 vehicles, 100% PEG 400 (Vehicle 1) and 10% HPBCD/1% $NaHCO_3$ in WFI (Vehicle 2) were administered (0.05 mL/10 g BW) concomitantly to mice in the vehicle group.

Tumor measurements:

Tumor size and body weight were measured BIW beginning on the first day of treatment. Animals were terminated when their tumor reached approximately 2000 $mm^3$, and the study was terminated on Day 62 post treatment initiation.

Inhibition of tumor growth was determined by calculating the percent TGI (MTV of the vehicle group MTV of a treated group) / MTV of the vehicle group on Day 20 post treatment initiation. Statistical comparisons of tumor growth between treatment groups and vehicle were conducted using a linear mixed effects regression analysis on the ΔAUC.

Statistical Analysis

The differences in the tumor growth trends over time between the vehicle control and treatment groups were assessed using linear mixed effects regression models. These models take into account that each animal was measured at multiple time points. A model was fit for the comparison, and the areas under the tumor volume-versus-time curve (AUCs) for control and treatment groups were calculated using the values predicted from the model. A statistically significant p value suggests that the trends over time for the two groups (vehicle and treatment) were different.

All tumor volumes had a value of 1 added to them before $log_{10}$ transformation. These values were compared across treatment groups to assess whether the differences in trends over time were statistically significant. To compare pairs of treatment groups, the following mixed-effects linear regression model was fit to the data using the maximum likelihood method:

$$Y_{ijk} - Y_{i0k} = Y_{i0k} + treat_i + day_j + day_j^2 + (treat * day)_{ij} + (treat * day^2)_{ij} + e_{ijk} \qquad (1)$$

where $Y_{ijk}$ is the $log_{10}$ tumor value at the $j^{th}$ time point of the $k^{th}$ animal in the $i^{th}$ treatment, $Y_{i0k}$ is the day 0 (baseline) $log_{10}$ tumor value in the $k^{th}$ animal in the $i^{th}$ treatment, $day_j$ was the median-centered time point and (along with $day^2_j$) was treated as a continuous variable, and $e_{ijk}$ is the residual error. A spatial power law covariance matrix was used to account for the repeated measurements on the same animal over time. Interaction terms as well as $day^2_j$ terms were removed if there were not statistically significant.

A likelihood ratio test was used to assess whether a given pair of treatment groups exhibited differences which were statistically significant. The -2 log likelihood of the full model was compared to one without any treatment terms (reduced model) and the difference in the values was tested using a Chi-squared test. The degrees of freedom of the test were calculated as the difference between the degrees of freedom of the full model and that of the reduced model.

The predicted differences in the log tumor values ($Y_{ijk}$-$Y_{i0k}$, which can be interpreted as $log_{10}$(fold change from day 0)) were taken from the above models to calculate mean AUC values for each treatment group. A dAUC value was then calculated as:

$$dAUC = \frac{mean(AUC_{ctl}) - mean(AUC_{trt})}{mean(AUC_{ctl})} * 100 \quad (2)$$

This assumed $AUC_{ctl}$ was positive. In instances where $AUC_{ctl}$ was negative, the above formula was multiplied by -1.

For synergy analysis, the observed differences in the log tumor values were used to calculate AUC values for each animal. In instances when an animal in a treatment group was removed from the study, the last observed tumor value was carried forward through all subsequent time points. The AUC for the control, or vehicle group was calculated using the predicted values from pairwise models described above. A measure of synergy was defined as follows:

$$Frac_{A_k} = \frac{AUC_{ctl} - AUC_{A_k}}{AUC_{ctl}} \quad (3)$$

$$Frac_{B_k} = \frac{AUC_{ctl} - AUC_{B_k}}{AUC_{ctl}} \quad (4)$$

$$Frac_{AB_k} = \frac{AUC_{ctl} - AUC_{AB_k}}{AUC_{ctl}} \quad (5)$$

$$\text{synergy score} = (mean(Frac_A) + mean(Frac_B) - mean(Frac_{AB})) * 100 \quad (6)$$

where $A_k$ and $B_k$ are the $k^{th}$ animal in the individual treatment groups and $AB_k$ is the $k^{th}$ animal in the combination treatment group. $AUC_{ctl}$ is the model-predicted AUC for the control group and was treated as a constant with no variability. The standard error of the synergy score was calculated as the square root of the sum of the squared standard erros across groups A, B, and AB. The degrees of freedom were estimated using the Welch-Satterthwaite equation. A hypothesis test was performed to determine if the synergy score differed from 0. P values were calculated by dividing the synergy score by its standard error and tested against a t-distribution (two-tailed) with the above-calculated degrees of freedom.

The effect was classified into four different categories. It was considered synergistic if the synergy score was less than 0 and additive if the synergy score wasn't statistically different from 0. If the synergy score was greater than zero, but the mean AUC for the combination was lower than the lowest mean AUC among the two single agent treatments, then the combination was sub-additive. If the synergy score was greater than zero, and the mean AUC for the combination was greater than the mean AUC for at least one of the single agent treatments, then the combination was antagonistic.

Interval analysis, if requested, involved a specified treatment group and time interval compared with another treatment group and time interval. For a given group, time interval, and animal, the tumor growth rate per day was estimated by

$$\text{Rate} = 100 * (10^{\Delta Y/\Delta t} - 1) \quad (7)$$

where $\Delta Y$ is the difference in the log 10 tumor volume over the interval of interest, and $\Delta t$ is the length of the time interval. If one or both of the time points were missing, then the animal was ignored. The mean rates across the animals were then compared to using a two-sided unpaired t-test with unequal variances. There were no adjustments pre-specified for the multiple comparisons and endpoints examined. All P values <0.05 were called statistically significant. Synergistic analysis: p> 0.05 = additive; p<0.05 and score <0 = synergistic; p<0.05, score >0, and the combination growth rate is lower than both the single agent growth rates = subadditive; p<0.05, score >0, and the combination growth rate is higher than at least one of the single agent growth rates = antagonistic.

Results

Combination treatment with Compound A (12.5 mg/kg QD or 50 mg/kg BIW) and docetaxel (IV, 7.5 mg/kg QW; TGI = 92.7% or 91.3%, respectively) resulted in a therapeutic advantage over single agent therapy and the interaction

of the two agents was additive. In the combination group treated with Compound A at 12.5 mg/kg QD and docetaxel at 7.5 mg/kg Q, there were two complete tumor regressions with one animal remaining tumor-free at study termination. The detail for this study is shown below in Table 2.

**Table 2. Summary of Results (N-Ras mutated SK-MEL-2 human melanoma)**

| Treatment Group | Dose (mg/ kg) | Method of Administration/ Frequency | Sex/No. Per Group | Species/ Strain | Endpoints | Findings |
|---|---|---|---|---|---|---|
| Vehicle 1 + Vehicle 2 | 0.0 | PO/QD<br>Days 0-20 | F/8 | Mouse (*Mus musculus*)Athymic NCr-*nu*/*nu* | TGI[a] | N/A |
| | | | | | Maximum Mean %BWL[b] | 0% |
| Compound A + Vehicle 2 | 12.5 | PO/QD<br>Days 0-20 | F/8 | Mouse (*Mus musculus*/ Athymic NCr-*nu*/*nu* | TGI | 70.7% |
| | | | | | ΔAUC | $p < 0.001$ |
| | | | | | Mean Maximum BWL[b] | 1 % (Days 3 and 10) |
| Compound A + Vehicle 2 | 50 | PO / BIW<br>Days 0, 3, 7, 10, 14, and 17 | F/8 | Mouse (*Mus musculus*/ Athymic NCr-*nulnu* | TGI | 62.9% |
| | | | | | ΔAUC | $p < 0.001$ |
| | | | | | Mean Maximum BWL[b] | 4% (Day 17) |
| | | | | | ΔAUC | $p < 0.001$ |
| | | | | | Mean Maximum BWL[b] | 4% (Day 14) |
| docetaxel + Vehicle 1 | 7.5 | IV/QW<br>Days 0, 7, and 14 | F/8 | Mouse (*Mus musculus*/ Athymic NCr-*nu*/*nu* | TGI | 74.9% |
| | | | | | ΔAUC | $p < 0.001$ |
| | | | | | Mean Maximum BWL[b] | 15% (Day 14) |
| | | | | | ΔAUC[c] | $p < 0.001$ |
| | | | | | Synergy analysis[d] | Additive p = 0.524 |
| | | | | | Mean Maximum BWL[b] | 7% (Day 14) |
| | | | | | Synergy analysis[d] | Subadditive p = 0.042 |
| | | | | | Mean Maximum BWL[b] | 9% (Day 14) |
| | | | | | ΔAUC[c] | $p < 0.001$ |
| | | | | | Synergy analysis[d] | Additive p = 0.404 |
| | | | | | Mean Maximum BWL[b] | 5% (Day 14) |

(continued)

| Treatment Group | Dose (mg/kg) | Method of Administration/ Frequency | Sex/No. Per Group | Species/ Strain | Endpoints | Findings |
|---|---|---|---|---|---|---|
| Compound A + docetaxel | 12.5 | PO/QD Days 0-20 | F/8 | Mouse (*Mus musculus*/ Athymic NCr-*nu*/*nu* | TGI | 92.7% |
| | | | | | ΔAUC[c] | $p < 0.001$ |
| | 7.5 | IV/QW Days 0, 7, and 14 | | | Synergy analysis[d] | Additive p = 0.344 |
| | | | | | Mean Maximum BWL[b] | 10% (Day 14) |
| Compound A + docetaxel | 50 | PO / BIW Days 0, 3, 7, 10, 14, and 17 | F/8 | Mouse (*Mus musculus*/ Athymic NCr-*nu*/*nu* | TGI | 91.3% |
| | | | | | ΔAUC[c] | $P < 0.001$ |
| | 7.5 | IV/QW Days 0, 7, and 14 | | | Synergy analysis[d] | Additive p = 0.252 |
| | | | | | Mean Maximum BWL[b] | 16% (Day 17) |

a Dose volume for each vehicle was 0.05 mL/10g BW

b TGI values were calculated on Day 20 post treatment initiation.

c Maximum mean percent BWL.

d ΔAUC = Statistical analysis was performed with a linear mixed effects regression model. A p value of < 0.05 was considered significant.

e Calculated on Day 20 post treatment initiation.

**Example 3: Methods for Measuring Markers**

B-Raf PCR based Assay (Vendor: Qiagen; Catalog#: 870801)

The B-Raf RGQ PCR Kit v2 combines two technologies, ARMS® and Scorpions®, to detect mutations in real-time PCR assays. This assay detects B-Raf V600 mutations V600E (GAG) and V600E complex (GAA), V600D (GAT), V600K (AAG), V600R (AGG). The kit detects the presence of the V600E (GAG) and V600E complex (GAA) but does not distinguish between them.

ARMS

Specific mutated sequences are selectively amplified by allele specific primer designed to match a mutated DNA.

Scorpions

Detection of amplification is performed using Scorpions. Scorpions are PCR primer covalently linked to a fluorescently labeled probe (i.e. FAM™ or HEX™) and a quencher. During PCR when the probe is bound to the amplicon, the fluorophore and quencher become separated resulting in an increase in fluorescence signal.

Procedure

The B-Raf RGQ PCR Kit v2 comprises a two-step procedure. In the first step, the control assay is performed to assess the total amplifiable B-Raf DNA in a sample. In the second step, both the mutation and control assays are performed to determine the presence or absence of mutant DNA.

• Control assay

The control assay, labeled with FAM, is used to assess the total amplifiable B-Raf DNA in a sample. The control assay amplifies a region of exon 3 of the B-Raf gene. The primers and Scorpion probe are designed to amplify independently of any known B-Raf polymorphisms.

• Mutation assays

Each mutation assay contains a FAM-labeled Scorpion probe and an ARMS primer for discrimination between the wild-type DNA and a specific mutant DNA.

Data Analysis: ΔCt Method

Scorpions real-time assays uses the number of PCR cycles necessary to detect a fluorescent signal above a background signal as a measure of the target molecules present at the beginning of the reaction. The point at which the signal is detected above background fluorescence is called the 'cycle threshold' (Ct).

Sample ΔCt values are calculated as the difference between the mutation assay Ct and control assay Ct from the same sample. Samples are classed as mutation positive if they give a ΔCt less than the Cut-Off ΔCt value for that assay. Above this value, the sample either contains less than the percentage of mutation able to be detected by the kit (beyond the limit of the assays), or the sample is mutation negative.

When using ARMS primers some inefficient priming could occur, giving a very late background Ct from DNA not containing a mutation. All ΔCt values calculated from background amplification are greater than the cut off ΔCt values and the sample is classed mutation negative.

For each sample, the ΔCt values are calculated as follows, ensuring that the mutation and control Ct values are from the same sample:

$$\Delta Ct = \{\text{sample mutation Ct}\} - \{\text{sample control Ct}\}$$

Sample control Ct can range between 27-33
Sample mutation Ct can range between 15-40
Acceptable ΔCt for the mutant call is <6 or 7

Methods for measuring N-Ras mutations are similar to those described above for B-Raf. Qiagen N-Ras assay for the detection of N-Ras Q61 mutations includes:

Q61K (181 C>A)
Q61R (182 A>G)

Methods for measuring K-Ras mutations are readily available. For example, Qiagen TheraScreen® K-Ras mutation kit.

**Example 4: Compound A and docetaxel combination in KRAS mutant lung cancer models**

Compound A and docetaxel combination was evaluated in KRAS mutant lung cancer models including Calu-6 lung cancer model ($KRAS^{Q61K}$) and A549 lung cancer model ($KRAS^{G12S}$). The results of the Calu-6 lung cancer model are described below. In the A549 model, the combination showed no better activity than single agents.
For the Calu-6 model, Compound A was formulated 100% PEG400 as follows:

1. Compound A at 10.0 mg/mL: 4.0 mL

- Weighed out 40.40 mg of Compound A powder;
- Added the powder in 4 mL of 100% PEG400;
- Sonicated until no Compound A particles remained and a solution was achieved;
- Stored at room temperature until use.

2. Compound A at 2.5 mg/mL: 20.0 mL

- Weighed out 50.51 mg of Compound A powder;
- Added the powder in 20 mL of 100% PEG400;
- Sonicated until no drug particles remained and a solution was achieved;
- Stored at room temperature until use.

Docetaxel (e.g., Taxotere Injection Concentrate, Sanofi-Aventis U.S. LLC) was formulated in 0.9% saline as follows:

1. Taxotere at 0.75 mg/mL: 8 mL

- 0.3 mL of Taxotere Injection Concentrate (20 mg/mL) was added into a centrifuge tube;
- Added 7.7 mL normal saline and mixed manually;
- Kept the tube protected from light and used within 4 hours.

2. Taxotere at 0.50 mg/mL: 6 mL

- 0.15 mL of Taxotere Injection Concentrate (20 mg/mL) was added into a centrifuge tube;
- Added 5.85 mL normal saline and mixed manually;
- Kept the tube protected from light and used within 4 hours.

Taxotere Injection Concentrate, instead of docetaxel, was used in recording raw data.

Animals in the vehicle treatment group were given 100% PEG400. The dose volume for vehicle or compound was 5 mL/kg body weight for PO and 10 mL/kg for IV administration.

The animals used in this study were Balb/c nude female mice (Shaghai SINO-British SIPPR/BK Lab Animal Ltd). The number of animals per group was 7. The age of the mice at Day 0 was 9 weeks. Group average weight at day 0 was 20.1 - 21.5 g.

Table 3 shows the dosing regimens for each treatment group used in the study.

**Table 3. Summary of Results (KRAS mutated Calu-6 human lung carcinoma).**

| **Treatment Group** | **Dose (mg/kg)** | **Method of Administration / Frequency** | **Sex/ Number Per Group** | **Species/ Strain** | **Endpoints** | **Findings** |
|---|---|---|---|---|---|---|
| Vehicle [a] | 0.0 | PO/QD Days 1-21 | F/7 | Mouse *(Mus musculus)* Athymic Balb/c Nude | TGI [b] | N/A |
| | | | | | Mean Maximum %BWL [c] | 0% |
| Compound A | 12.5 | PO/QD Days 1-21 | F/7 | Mouse *(Mus musculus)* Athymic Balb/c Nude | TGI[b] | 58.9% |
| | | | | | $\Delta$AUC[d] | 55.4 ($p < 0.001$) |
| | | | | | Mean Maximum %BWL [c] | 0% |
| Compound A | 50 | PO / BIW Days 1,4,8,11, | F/7 | Mouse (*Mus musculus*) | TGI[b] | 59.4% |
| | | | | | $\Delta$AUC[d] | 62.7 ($p < 0.001$) |

(continued)

| **Treatment Group** | **Dose (mg/kg)** | **Method of Administration / Frequency** | **Sex/ Number Per Group** | **Species/ Strain** | **Endpoints** | **Findings** |
|---|---|---|---|---|---|---|
| | | 15, and 18 | | Athymic Balb/c Nude | Mean Maximum %BWL[c] | -0.1% (Day 7) |
| | | | | | ΔAUC[d] | 29.7 ($p < 0.01$) |
| | | | | | Mean Maximum %BWL[c] | 0% |
| docetaxel | 5 | IV / QW Days 1, 8, and 15 | F/7 | Mouse (*Mus musculus)* Athymic Balb/c Nude | TGI[b] | 5.2% |
| | | | | | ΔAUC[d] | 9.0 ($p > 0.05$) |
| | | | | | Mean Maximum %BWL[c] | 0% |
| docetaxel | 7.5 | IV / QW Days 1, 8, and 15 | F/7 | Mouse (*Mus musculus*) Athymic Balb/c Nude | TGI[b] | 17.7% |
| | | | | | ΔAUC[d] | 18.4 ($p < 0.05$) |
| | | | | | Mean Maximum %BWL[c] | 2.5% (Day 21) |
| Compound A + docetaxel | 12.5 5 | PO / QD Days 1-21 IV / QW Days 1, 8, and 15 | F/7 | Mouse (*Mus musculus*) Athymic Balb/c Nude | TGI[b] | 69.1% |
| | | | | | ΔAUC[d] | 71.3 ($p < 0.001$) |
| | | | | | Synergy analysis[e] | Additive ($p = 0.614$) |
| | | | | | Mean Maximum %BWL [c] | 8.9% (Day 21) |
| Compound A + docetaxel | 12.5<br>7.5 | PO / QD Days 1-21 IV / QW Days 1, 8, and 15 | F/7 | Mouse *(Mus musculus)* Athymic Balb/c Nude | TGI[b] | 69.4% |
| | | | | | ΔAUC[d] | 75.4 ($p < 0.001$) |
| | | | | | Synergy analysis[e] | Additive ($p = 0.926$) |
| | | | | | Mean Maximum %BWL[c] | 14.0% (Day 21) |

(continued)

| Treatment Group | Dose (mg/kg) | Method of Administration / Frequency | Sex/ Number Per Group | Species/ Strain | Endpoints | Findings |
|---|---|---|---|---|---|---|
| Compound A + docetaxel | 50<br><br>7.5 | PO/BIW Days 1, 4, 8, 11, 15, and 18 IV / QW Days 1, 8, and 15 | F/7 | Mouse (*Mus musculus)* Athymic Balb/c Nude | TGI[b] | 63.9% |
| | | | | | ΔAUC[d] | 71.5 ($p < 0.001$) |
| | | | | | Synergy analysis[e] | Additive ($p = 0.360$) |
| | | | | | Mean Maximum %BWL[c] | 8.0% (Day 21) |

ΔAUC = change in areas under the tumor volume-versus-time curves; BIW = twice weekly; BWL = body weight loss; IV = intravenously; N/A = not applicable; QD = daily; QW = weekly; TGI = tumor growth inhibition; Vehicle = 100% PEG400.

[a] Dose volume for vehicle or compound was 5 mL/kg body weight for PO and 10 mL/kg for IV administration.
[b] TGI values were calculated on Day 21 post treatment initiation.
[c] Maximum mean percent BWL between Day 0 to Day 21.
[d] ΔAUC = Statistical analysis was performed with a linear mixed effects regression model. A p value of < 0.05 was considered significant.
[e] Calculated on Day 21 post treatment initiation.

Each animal was inoculated with 2 x $10^6$ Calu-6 tumor cells (in 0.1 mL, 1:1 with Matrigel) at the right flank for tumor model development. Body weight and the tumor growth were monitored twice weekly. Tumor size was measured to the nearest 0.1 mm using vernier caliper and applying the formula $V = W^2 \times L/2$, where V = volume, W = width, and L = length for the tumor xenograft. Xenografts were allowed to grow until they reached an average size of approximately 220 $mm^3$ after 14 days. Mice bearing the proper size xenograft were randomly assigned into one of the groups shown in the table above and began to be treated with their assigned test materials, either vehicle (100% PEG400), Compound A (at 12.5 or 50 mg/kg), docetaxel (at 5 or 7.5 mg/kg), or the combination of Compound A/docetaxel.

Tumor size and body weight were measured twice weekly beginning on the day of animal grouping. The study was terminated on Day 35 post treatment initiation.

Inhibition of tumor growth was determined by calculating the percent TGI (MTV of the vehicle group - MTV of a treated group) / MTV of the vehicle group] on Day 21 of the study. Statistical comparisons of tumor growth between treatment groups and vehicle were conducted using a linear mixed effects regression analysis on the ΔAUC.

Statistical Tests

Delta AUC

The differences in the tumor growth trends over time between the vehicle control and treatment groups were assessed using linear mixed effects regression models. These models take into account that each animal was measured at multiple time points. A model was fit for the comparison, and the areas under the tumor volume-versus-time curve (AUCs) for control and treatment groups were calculated using the values predicted from the model. A statistically significant p value suggests that the trends over time for the 2 groups (vehicle and treatment) were different. A p value <0.05 was considered statistically significant. Further details regarding pair-wise comparisons are provided Example 2.

A combination score calculation was used to address the question of whether the effects of the combination treatments were synergistic, additive, subadditive, or antagonistic relative to the individual treatments. The effect was considered synergistic if the synergy score was less than 0, and additive if the synergy score wasn't statistically different from 0. If the synergy score was greater than 0, but the mean AUC for the combination was lower than the lowest mean AUC among the two single agent treatments, then the combination was subadditive. If the synergy score was greater

than zero, and the mean AUC for the combination was greater than the mean AUC for at least one of the single agent treatments, then the combination was antagonistic.

## Claims

1. A Raf kinase inhibitor, or a pharmaceutically acceptable salt thereof, for use in the treatment of cancer, in combination with a taxane, or a pharmaceutically acceptable salt thereof, wherein the Raf kinase inhibitor is

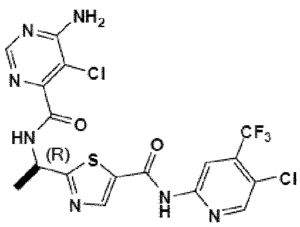

(Compound A); and

the taxane is docetaxel (taxotere).

2. The Raf kinase inhibitor, or pharmaceutically acceptable salt thereof, for use of claim 1, wherein the cancer is a K-Ras exon 2 mutation positive cancer.

3. The Raf kinase inhibitor, or pharmaceutically acceptable salt thereof, for use of any one of claims 1 or 2, wherein the cancer is a B-Raf mutation positive cancer.

4. The Raf kinase inhibitor, or pharmaceutically acceptable salt thereof, for use of claim 3, wherein the cancer is a non-V600 B-Raf mutation positive cancer.

5. The Raf kinase inhibitor, or pharmaceutically acceptable salt thereof, for use of any one of claims 1-4, wherein the cancer is selected from the group consisting of skin cancer, ocular cancer, gastrointestinal cancer, thyroid cancer, breast cancer, ovarian cancer, central nervous system cancer, laryngeal cancer, cervical cancer, lymphatic system

cancer, genitourinary tract cancer, bone cancer, biliary tract cancer, endometrial cancer, uterine cancer, liver cancer, lung cancer, prostate cancer, and colon cancer.

**6.** The Raf kinase inhibitor, or pharmaceutically acceptable salt thereof, for use of claim 1, wherein the cancer is non-small cell lung cancer.

**7.** The Raf kinase inhibitor, or pharmaceutically acceptable salt thereof, for use of claim 6, wherein the non-small cell lung cancer is a K-Ras mutation positive cancer.

**8.** The Raf kinase inhibitor, or pharmaceutically acceptable salt thereof, for use of claim 6, wherein the non-small cell lung cancer is a B-Raf mutation positive cancer.

**9.** The Raf kinase inhibitor, or pharmaceutically acceptable salt thereof, for use any one of claims 1-8, wherein Compound A, or a pharmaceutically acceptable salt thereof, is administered once weekly (QW) with a rest period of 6 days between each administration in an amount of up to 600 mg per dose.

**10.** The Raf kinase inhibitor, or pharmaceutically acceptable salt thereof, for use of claim 9, wherein Compound A, or a pharmaceutically acceptable salt thereof, is administered in an amount of about 400 mg to about 600 mg per dose.

**11.** A pharmaceutical composition comprising:

(i) a Raf kinase inhibitor, or a pharmaceutically acceptable salt thereof; and
(ii) a taxane, or a pharmaceutically acceptable salt thereof;

wherein the Raf kinase inhibitor is

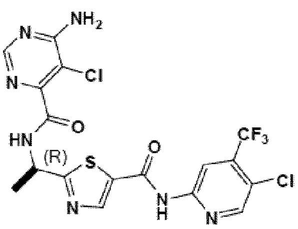

(Compound A); and

the taxane is docetaxel (taxotere).

**Patentansprüche**

**1.** Raf-Kinase-Inhibitor oder pharmazeutisch unbedenkliches Salz davon zur Verwendung bei der Behandlung von Krebs in Kombination mit einem Taxan oder einem pharmazeutisch unbedenklichen Salz davon, wobei es sich bei dem Raf-Kinase-Inhibitor um

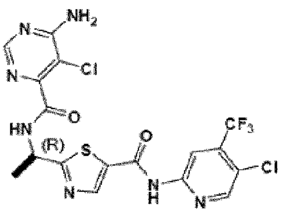

(Verbindung A)

handelt und

es sich bei dem Taxan um Docetaxel (Taxotere) handelt.

2. Raf-Kinase-Inhibitor oder pharmazeutisch unbedenkliches Salz davon zur Verwendung nach Anspruch 1, wobei es sich bei dem Krebs um einen K-Ras-Exon-2-mutationspositiven Krebs handelt.

3. Raf-Kinase-Inhibitor oder pharmazeutisch unbedenkliches Salz davon zur Verwendung nach Anspruch 1 oder 2, wobei es sich bei dem Krebs um einen B-Raf-mutationspositiven Krebs handelt.

4. Raf-Kinase-Inhibitor oder pharmazeutisch unbedenkliches Salz davon zur Verwendung nach Anspruch 3, wobei es sich bei dem Krebs um einen Nicht-V600-B-Raf-mutationspositiven Krebs handelt.

5. Raf-Kinase-Inhibitor oder pharmazeutisch unbedenkliches Salz davon zur Verwendung nach einem der Ansprüche 1-4, wobei der Krebs aus der Gruppe bestehend aus Hautkrebs, Augenkrebs, Magen-Darm-Krebs, Schilddrüsenkrebs, Brustkrebs, Eierstockkrebs, Zentralnervensystemkrebs, Kehlkopfkrebs, Gebärmutterhalskrebs, Lymphsystemkrebs, Urogenitalsystemkrebs, Knochenkrebs, Gallenwegskrebs, Endometriumkrebs, Gebärmutterkrebs, Leberkrebs, Lungenkrebs, Prostatakrebs und Kolonkrebs ausgewählt ist.

6. Raf-Kinase-Inhibitor oder pharmazeutisch unbedenkliches Salz davon zur Verwendung nach Anspruch 1, wobei es sich bei dem Krebs um nichtkleinzelligen Lungenkrebs handelt.

7. Raf-Kinase-Inhibitor oder pharmazeutisch unbedenkliches Salz davon zur Verwendung nach Anspruch 6, wobei es sich bei dem nichtkleinzelligen Lungenkrebs um einen K-Rasmutationspositiven Krebs handelt.

8. Raf-Kinase-Inhibitor oder pharmazeutisch unbedenkliches Salz davon zur Verwendung nach Anspruch 6, wobei es sich bei dem nichtkleinzelligen Lungenkrebs um einen B-Raf-mutationspositiven Krebs handelt.

9. Raf-Kinase-Inhibitor oder pharmazeutisch unbedenkliches Salz davon zur Verwendung nach einem der Ansprüche 1-8, wobei Verbindung A oder ein pharmazeutisch unbedenkliches Salz davon einmal wöchentlich (QW) mit einem Zeitraum von 6 Tagen zwischen jeder Verabreichung in einer Menge von bis zu 600 mg pro Dosis verabreicht wird.

10. Raf-Kinase-Inhibitor oder pharmazeutisch unbedenkliches Salz davon zur Verwendung nach Anspruch 9, wobei Verbindung A oder ein pharmazeutisch unbedenkliches Salz davon in einer Menge von etwa 400 mg bis etwa 600 mg pro Dosis verabreicht wird.

11. Pharmazeutische Zusammensetzung, umfassend:

(i) einen Raf-Kinase-Inhibitor oder ein pharmazeutisch unbedenkliches Salz davon und
(ii) ein Taxan oder ein pharmazeutisch unbedenkliches Salz davon;

wobei sich bei dem Raf-Kinase-Inhibitor um

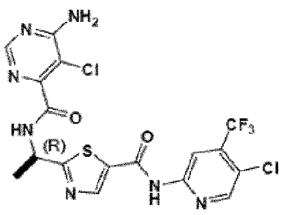

(Verbindung A)

handelt und
es sich bei dem Taxan um Docetaxel (Taxotere) handelt.

## Revendications

1. Inhibiteur de la kinase Raf, ou sel pharmaceutiquement acceptable correspondant, pour une utilisation dans le

traitement du cancer, en combinaison avec un taxane, ou un sel pharmaceutiquement acceptable correspondant, l'inhibiteur de kinase Raf étant

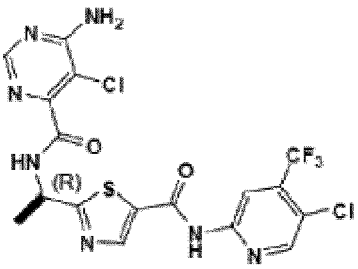

(Composé A) ; et

le taxane étant le docétaxel (taxotère).

**2.** Inhibiteur de la kinase Raf, ou sel pharmaceutiquement acceptable correspondant, pour une utilisation selon la revendication 1, le cancer étant un cancer présentant une mutation de l'exon 2 de la K-Ras.

**3.** Inhibiteur de la kinase Raf, ou sel pharmaceutiquement acceptable correspondant, pour une utilisation selon l'une quelconque des revendications 1 et 2, le cancer étant un cancer présentant une mutation de la B-Raf.

**4.** Inhibiteur de la kinase Raf, ou sel pharmaceutiquement acceptable correspondant, pour une utilisation selon la revendication 3, le cancer étant un cancer présentant une mutation de B-Raf non V600.

**5.** Inhibiteur de la kinase Raf, ou sel pharmaceutiquement acceptable correspondant, pour une utilisation selon l'une quelconque des revendications 1 à 4, le cancer étant choisi dans le groupe constitué par le cancer de la peau, le cancer oculaire, le cancer gastro-intestinal, le cancer de la thyroïde, le cancer du sein, le cancer des ovaires, le cancer du système nerveux central, le cancer du larynx, le cancer du col de l'utérus, le cancer du système lymphatique, le cancer du tract génito-urinaire, le cancer des os, le cancer du tract biliaire, le cancer endométrial, le cancer de l'utérus, le cancer du foie, le cancer du poumon, le cancer de la prostate, et le cancer du côlon.

**6.** Inhibiteur de la kinase Raf, ou sel pharmaceutiquement acceptable correspondant, pour une utilisation selon la revendication 1, le cancer étant le cancer du poumon non à petites cellules.

**7.** Inhibiteur de la kinase Raf, ou sel pharmaceutiquement acceptable correspondant, pour une utilisation selon la revendication 6, le cancer du poumon non à petites cellules étant un cancer présentant une mutation de la K-Ras.

**8.** Inhibiteur de la kinase Raf, ou sel pharmaceutiquement acceptable correspondant, pour une utilisation selon la revendication 6, le cancer du poumon non à petites cellules étant un cancer présentant une mutation de la B-Raf.

**9.** Inhibiteur de la kinase Raf, ou sel pharmaceutiquement acceptable correspondant, pour une utilisation selon l'une quelconque des revendications 1 à 8, le Composé A, ou un sel pharmaceutiquement acceptable correspondant, étant administré une fois par semaine (QW) avec une période de repos de 6 jours entre chaque administration en une quantité allant jusqu'à 600 mg par dose.

**10.** Inhibiteur de la kinase Raf, ou sel pharmaceutiquement acceptable correspondant, pour une utilisation selon la revendication 9, le Composé A, ou un sel pharmaceutiquement acceptable correspondant, étant administré en une quantité d'environ 400 mg à environ 600 mg par dose.

**11.** Composition pharmaceutique comprenant :

(i) un inhibiteur de kinase Raf, ou un sel pharmaceutiquement acceptable correspondant ; et
(ii) un taxane, ou un sel pharmaceutiquement acceptable correspondant ;

l'inhibiteur de kinase Raf étant

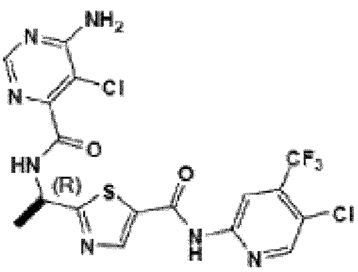

(Composé A) ; et

le taxane étant le docétaxel (taxotère).

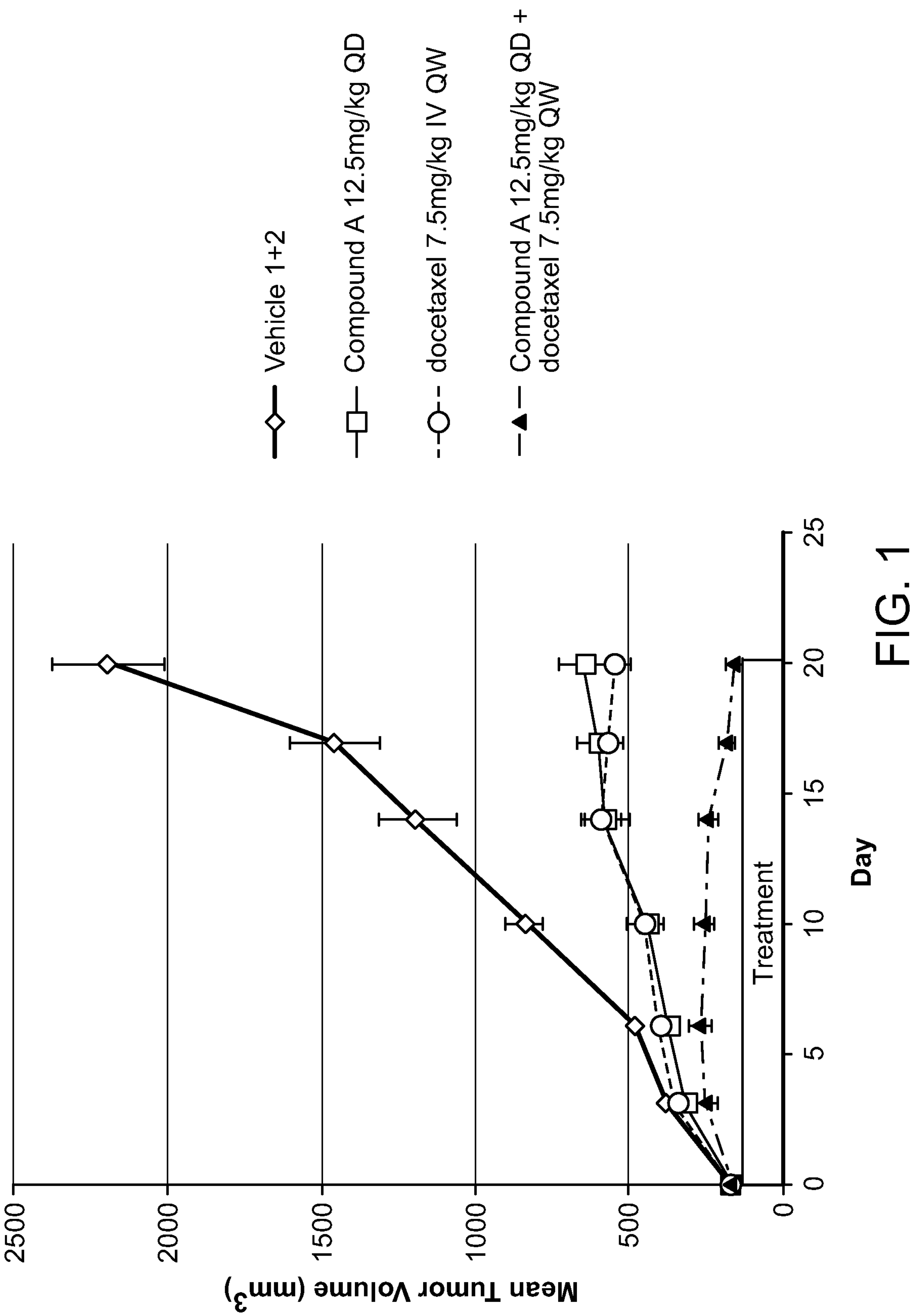
Mean Tumor Volume (mm3)
2500
2000
1500
1000
500
0
0
5
10
15
20
25
Day
Treatment
Vehicle 1+2
Compound A 12.5mg/kg QD
docetaxel 7.5mg/kg IV QW
Compound A 12.5mg/kg QD + docetaxel 7.5mg/kg QW

FIG. 1

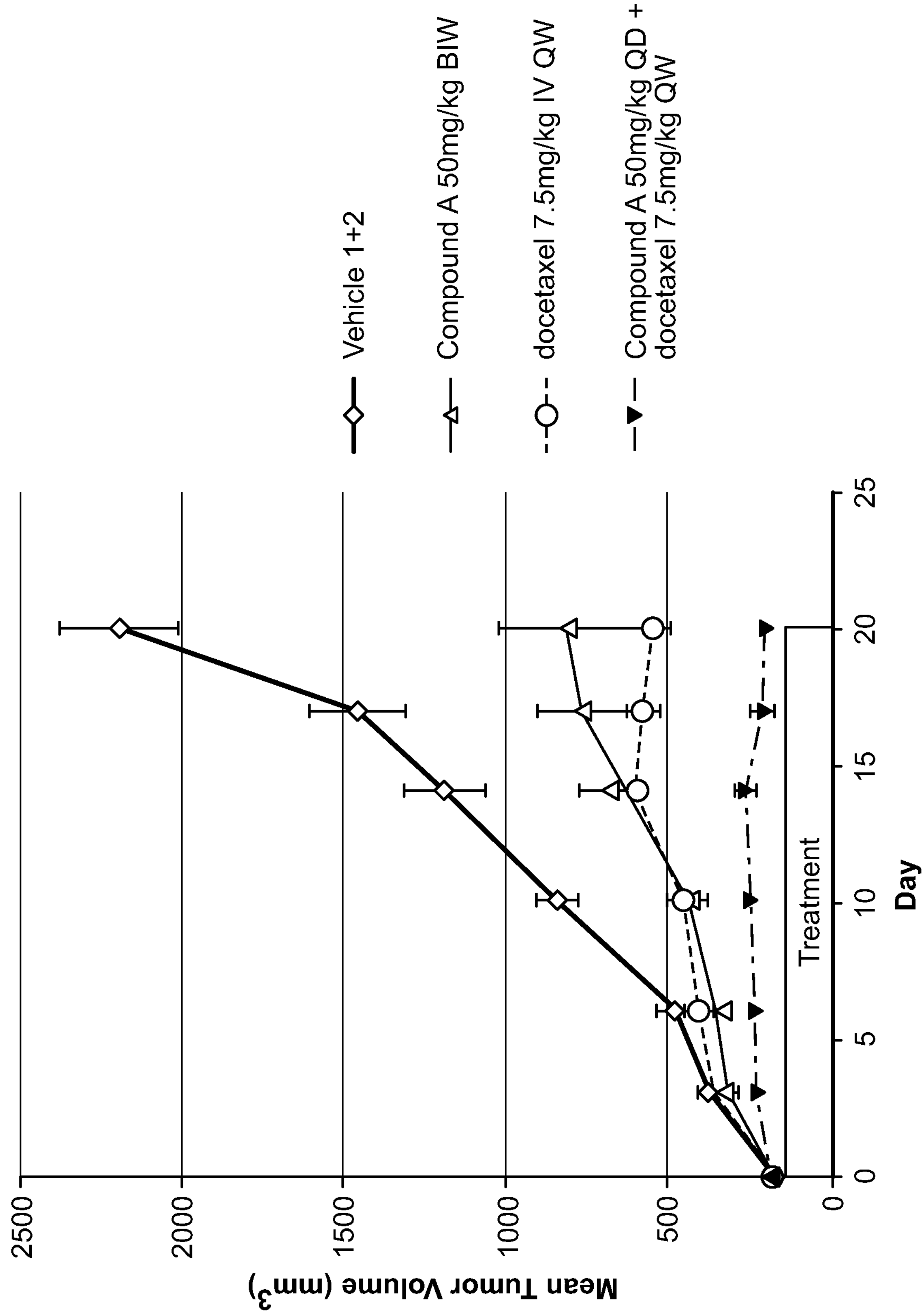
Mean Tumor Volume (mm$^3$)
0
500
1000
1500
2000
2500
Day
0
5
10
15
20
25
Treatment
Vehicle 1+2
Compound A 50mg/kg BIW
docetaxel 7.5mg/kg IV QW
Compound A 50mg/kg QD +
docetaxel 7.5mg/kg QW

FIG. 2

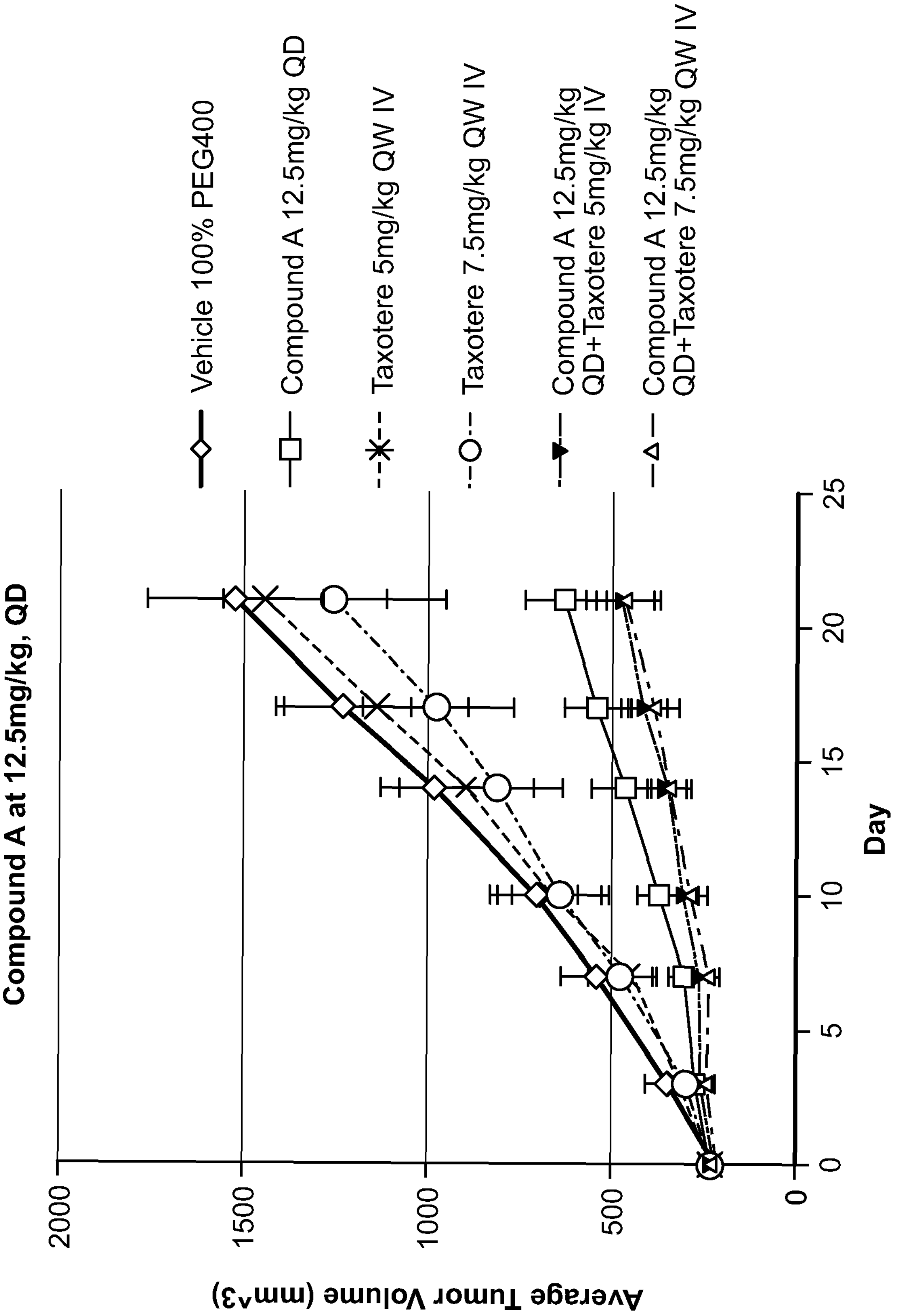
Compound A at 12.5mg/kg, QD
Average Tumor Volume (mm^3)
2000
1500
1000
500
0
0
5
10
15
20
25
Day
Vehicle 100% PEG400
Compound A 12.5mg/kg QD
Taxotere 5mg/kg QW IV
Taxotere 7.5mg/kg QW IV
Compound A 12.5mg/kg QD+Taxotere 5mg/kg IV
Compound A 12.5mg/kg QD+Taxotere 7.5mg/kg QW IV

FIG. 3

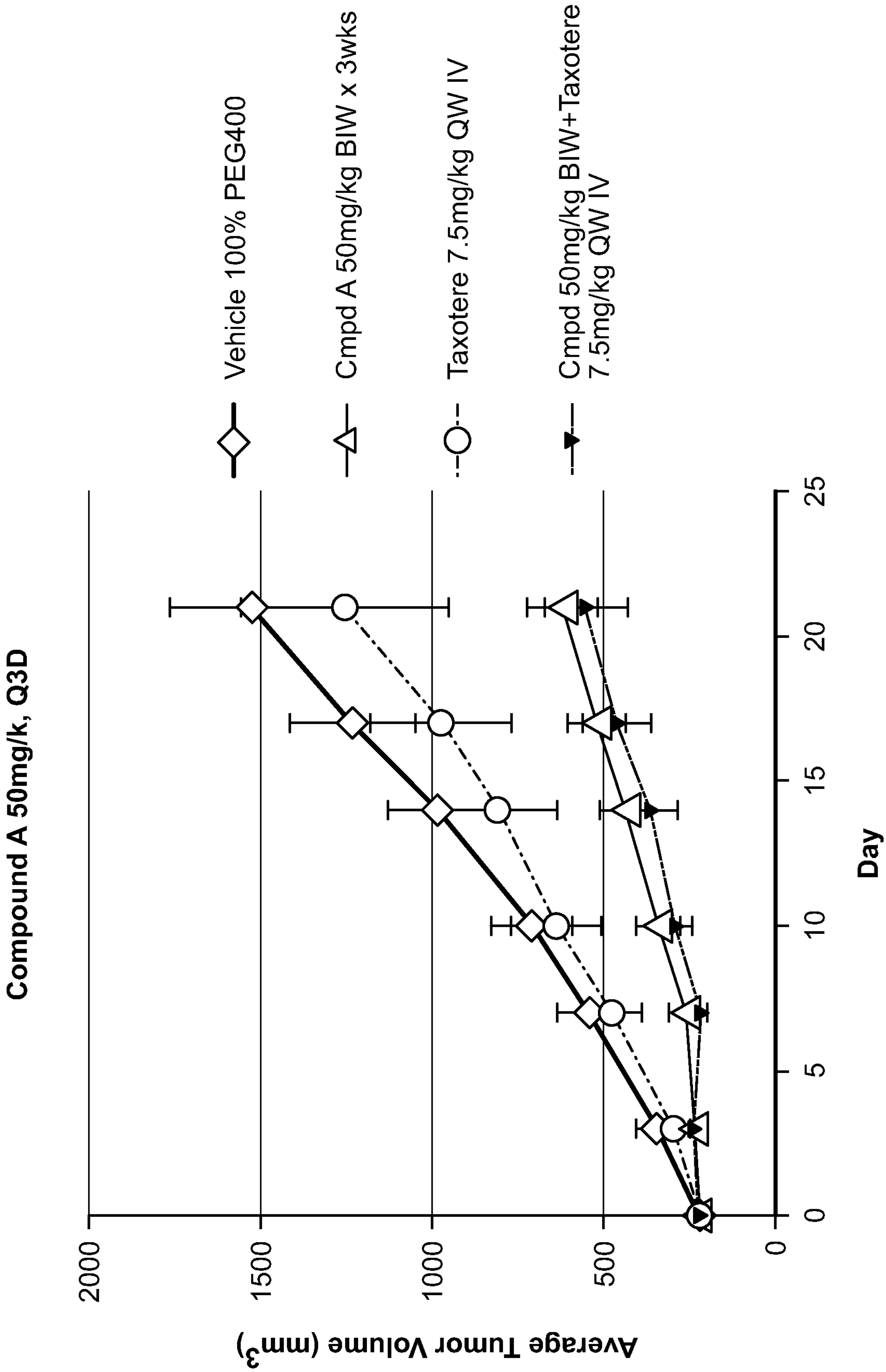
Compound A 50mg/k, Q3D
Average Tumor Volume (mm3)
2000
1500
1000
500
0
0
5
10
15
20
25
Day
Vehicle 100% PEG400
Cmpd A 50mg/kg BIW x 3wks
Taxotere 7.5mg/kg QW IV
Cmpd 50mg/kg BIW+Taxotere 7.5mg/kg QW IV

FIG. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 62096064 B
- US 62242629 B
- WO 2009006389 A
- WO 2010064722 A
- WO 2006065703 A
- WO 2011117381 A
- WO 2011090738 A
- WO 2011161216 A
- WO 2011097526 A
- WO 2011025927 A
- WO 2011023773 A
- WO 2011147764 A
- WO 2011079133 A
- WO 2011063159 A
- US 20130252977 A
- US 6096331 A
- US 6506405 B
- WO 2015022792 A
- WO 2015148828 A
- US 4843155 A
- US 5346994 A


### Non-patent literature cited in the description


- **BALANI et al.** *Eur. J. Cancer,* 2014, vol. 50 (6), 54-55
- **S. M. BERGE et al.** describe pharmaceutically acceptable salts in detail. *J. Pharmaceutical Sciences,* 1977, vol. 66, 1-19
- **FOTIADOU et al.** *Mol. Cel. Biol.,* 2007, vol. 27, 6742-6755
- **BERGE et al.** *J. Pharm. Sci.,* 1977, vol. 66, 1-19
- **A. GENNARO ; LIPPINCOTT WILLIAMS ; WILKINS.** *Remington: The Science and Practice of Pharmacy,* 2000
- Remington: The Science and Practice of Pharmacy. Lippincott Williams & Wilkins, 2000
- *Lancet,* 2012, vol. 380, 358-365
- *N Engl J Med,* 2012, vol. 367, 107-114
- **NAZARIAN et al.** *Nature,* 2010, vol. 468, 973-7
- **SU et al.** *New England Journal of Medicine,* 2012, vol. 366, 207-215
- **JOHANNESSEN et al.** *Nature,* 2010, vol. 468, 968-72
- **MONTAGUT et al.** *Cancer Res.,* 2008, vol. 68, 4853-61
- **POULIKAKOS et al.** *Nature,* 2011, vol. 480, 387-390
- **VILLANUEVA et al.** *Cancer Cell.,* 2010, vol. 18, 683-95
- **SHI et al.** *Cancer Res.,* 2011, vol. 71, 5067-74
- **HATZIVASSILIOU G et al.** *Nature,* 2010, vol. 464, 431-435
- **POULIKAKOS et al.** *Nature,* 2010, vol. 464, 427-430
- **HEIDORN et al.** *Cell,* 2010, vol. 140, 209-221
- **SCHUBBERT et al.** *Nature Reviews Cancer,* 2007, vol. 7, 295
- **PYLAYEVA-GUPTA et al.** *Nature Reviews Cancer,* 2011, vol. 11, 761
- **ZHANG et al.** *J. Genet. Genomics,* 2011, vol. 38, 95-109
- **SHENDURE ; HANLEE.** *Nature Biotech,* 2008, vol. 26, 1135-1145
- **AUSUBEL et al.** Current Protocols in Molecular Biology. John Wiley & Sons, 1987
- **CHOMCZYNSKI ; SACCHI.** *Anal. Biochem.,* 1987, vol. 162, 156-159
- **CHIRGWIN et al.** *Biochemistry,* 1979, vol. 18, 5294-99
- **SAMBROOK et al.** Molecular Cloning--A Laboratory Manual. Cold Spring Harbor Laboratory, 1989
- **AUSUBEL et al.** *Current Protocols In Molecular Biology,* 1994, vol. 2
- **MYERS et al.** *Science,* 1985, vol. 230, 1242
- **SAIKI et al.** *Nature,* 1986, vol. 324, 163
- **SAIKI et al.** *Proc. Natl Acad. Sci USA,* 1989, vol. 86, 6230
- **WALLACE et al.** *Nucl. Acids Res.,* 1979, vol. 6, 3543

SEQUENCE LISTING

<110> Bozon, Viviana Galvin, Katherine Brake, Rachel Millennium Pharmaceuticals, inc.

<120> COMBINATION OF RAF INHIBITORS AND TAXANES

<130> MPI14-028P2M

<160> 9

<170> FastSEQ for Windows Version 4.0

<210> 1
<211> 2949
<212> DNA
<213> Homo sapiens

<400> 1

cgcctccctt ccccctcccc gcccgacagc ggccgctcgg gccccggctc tcggttataa 60
gatggcggcg ctgagcggtg gcggtggtgg cggcgcggag ccgggccagg ctctgttcaa 120
cggggacatg gagcccgagg ccggcgccgg cgccggcgcc gcggcctctt cggctgcgga 180
ccctgccatt ccggaggagg tgtggaatat caaacaaatg attaagttga cacaggaaca 240
tatagaggcc ctattggaca aatttggtgg ggagcataat ccaccatcaa tatatctgga 300
ggcctatgaa gaatacacca gcaagctaga tgcactccaa caaagagaac aacagttatt 360
ggaatctctg gggaacggaa ctgatttttc tgtttctagc tctgcatcaa tggataccgt 420
tacatcttct tcctcttcta gcctttcagt gctaccttca tctctttcag tttttcaaaa 480
tcccacagat gtggcacgga gcaaccccaa gtcaccacaa aaacctatcg ttagagtctt 540
cctgcccaac aaacagagga cagtggtacc tgcaaggtgt ggagttacag tccgagacag 600
tctaaagaaa gcactgatga tgagaggtct aatcccagag tgctgtgctg tttacagaat 660
tcaggatgga gagaagaaac caattggttg ggacactgat atttcctggc ttactggaga 720
agaattgcat gtggaagtgt tggagaatgt tccacttaca acacacaact ttgtacgaaa 780
aacgtttttc accttagcat tttgtgactt ttgtcgaaag ctgcttttcc agggtttccg 840
ctgtcaaaca tgtggttata aatttcacca gcgttgtagt acagaagttc cactgatgtg 900
tgttaattat gaccaacttg atttgctgtt tgtctccaag ttctttgaac accacccaat 960
accacaggaa gaggcgtcct tagcagagac tgccctaaca tctggatcat ccccttccgc 1020
acccgcctcg gactctattg ggccccaaat tctcaccagt ccgtctcctt caaaatccat 1080
tccaattcca cagcccttcc gaccagcaga tgaagatcat cgaaatcaat ttgggcaacg 1140
agaccgatcc tcatcagctc ccaatgtgca tataaacaca atagaacctg tcaatattga 1200
tgacttgatt agagaccaag gatttcgtgg tgatggagga tcaaccacag gtttgtctgc 1260
tacccccct gcctcattac ctggctcact aactaacgtg aaagccttac agaaatctcc 1320
aggacctcag cgagaaagga agtcatcttc atcctcagaa gacaggaatc gaatgaaaac 1380
acttggtaga cgggactcga gtgatgattg ggagattcct gatgggcaga ttacagtggg 1440
acaaagaatt ggatctggat catttggaac agtctacaag ggaaagtggc atggtgatgt 1500
ggcagtgaaa atgttgaatg tgacagcacc tacacctcag cagttacaag ccttcaaaaa 1560
tgaagtagga gtactcagga aaacacgaca tgtgaatatc ctactcttca tgggctattc 1620
cacaaagcca caactggcta ttgttaccca gtggtgtgag ggctccagct tgtatcacca 1680
tctccatatc attgagacca aatttgagat gatcaaactt atagatattg cacgacagac 1740
tgcacagggc atggattact tacacgccaa gtcaatcatc cacagagacc tcaagagtaa 1800
taatatattt cttcatgaag acctcacagt aaaaataggt gattttggtc tagctacagt 1860
gaaatctcga tggagtgggt cccatcagtt tgaacagttg tctggatcca ttttgtggat 1920
ggcaccagaa gtcatcagaa tgcaagataa aaatccatac agctttcagt cagatgtata 1980
tgcatttgga attgttctgt atgaattgat gactggacag ttaccttatt caaacatcaa 2040
caacagggac cagataattt ttatggtggg acgaggatac ctgtctccag atctcagtaa 2100
ggtacggagt aactgtccaa aagccatgaa gagattaatg gcagagtgcc tcaaaaagaa 2160
aagagatgag agaccactct ttccccaaat tctcgcctct attgagctgc tggcccgctc 2220
attgccaaaa attcaccgca gtgcatcaga accctccttg aatcgggctg gtttccaaac 2280
agaggatttt agtctatatg cttgtgcttc tccaaaaaca cccatccagg caggggggata 2340

tggtgcgttt cctgtccact gaaacaaatg agtgagagag ttcaggagag tagcaacaaa 2400
aggaaaataa atgaacatat gtttgcttat atgttaaatt gaataaaata ctctcttttt 2460
ttttaaggtg aaccaaagaa cacttgtgtg gttaaagact agatataatt tttccccaaa 2520
ctaaaattta tacttaacat tggattttta acatccaagg gttaaaatac atagacattg 2580
ctaaaaattg gcagagcctc ttctagaggc tttactttct gttccgggtt tgtatcattc 2640
acttggttat tttaagtagt aaacttcagt ttctcatgca acttttgttg ccagctatca 2700
catgtccact agggactcca gaagaagacc ctacctatgc ctgtgtttgc aggtgagaag 2760
ttggcagtcg gttagcctgg gttagataag gcaaactgaa cagatctaat ttaggaagtc 2820
agtagaattt aataattcta ttattattct taataatttt tctataacta tttcttttta 2880
taacaatttg gaaaatgtgg atgtctttta tttccttgaa gcaataaact aagtttcttt 2940
ttataaaaa 2949

<210> 2
<211> 2301
<212> DNA
<213> Homo sapiens

<400> 2

```
atggcggcgc tgagcggtgg cggtggtggc ggcgcggagc cgggccaggc tctgttcaac 60
ggggacatgg agcccgaggc cggcgccggc gccggcgccg cggcctcttc ggctgcggac 120
cctgccattc cggaggaggt gtggaatatc aaacaaatga ttaagttgac acaggaacat 180
atagaggccc tattggacaa atttggtggg gagcataatc caccatcaat atatctggag 240
gcctatgaag aatacaccag caagctagat gcactccaac aaagagaaca acagttattg 300
gaatctctgg ggaacggaac tgatttttct gtttctagct ctgcatcaat ggataccgtt 360
acatcttctt cctcttctag cctttcagtg ctaccttcat ctctttcagt ttttcaaaat 420
cccacagatg tggcacggag caaccccaag tcaccacaaa aacctatcgt tagagtcttc 480
ctgcccaaca aacagaggac agtggtacct gcaaggtgtg gagttacagt ccgagacagt 540
ctaaagaaag cactgatgat gagaggtcta atcccagagt gctgtgctgt ttacagaatt 600
caggatggag agaagaaacc aattggttgg gacactgata tttcctggct tactggagaa 660
gaattgcatg tggaagtgtt ggagaatgtt ccacttacaa cacacaactt tgtacgaaaa 720
acgtttttca ccttagcatt ttgtgacttt tgtcgaaagc tgcttttcca gggtttccgc 780
tgtcaaacat gtggttataa atttcaccag cgttgtagta cagaagttcc actgatgtgt 840
gttaattatg accaacttga tttgctgttt gtctccaagt tctttgaaca ccacccaata 900
ccacaggaag aggcgtcctt agcagagact gccctaacat ctggatcatc cccttccgca 960
cccgcctcgg actctattgg gccccaaatt ctcaccagtc cgtctccttc aaaatccatt 1020
ccaattccac agcccttccg accagcagat gaagatcatc gaaatcaatt tgggcaacga 1080
gaccgatcct catcagctcc caatgtgcat ataaacacaa tagaacctgt caatattgat 1140
gacttgatta gagaccaagg atttcgtggt gatggaggat caaccacagg tttgtctgct 1200
acccccccctg cctcattacc tggctcacta actaacgtga aagccttaca gaaatctcca 1260
ggacctcagc gagaaaggaa gtcatcttca tcctcagaag acaggaatcg aatgaaaaca 1320
cttggtagac gggactcgag tgatgattgg gagattcctg atgggcagat tacagtggga 1380
caaagaattg gatctggatc atttggaaca gtctacaagg gaaagtggca tggtgatgtg 1440
gcagtgaaaa tgttgaatgt gacagcacct acacctcagc agttacaagc cttcaaaaat 1500
gaagtaggag tactcaggaa aacacgacat gtgaatatcc tactcttcat gggctattcc 1560
acaaagccac aactggctat tgttacccag tggtgtgagg gctccagctt gtatcaccat 1620
ctccatatca ttgagaccaa atttgagatg atcaaactta tagatattgc acgacagact 1680
gcacagggca tggattactt acacgccaag tcaatcatcc acagagacct caagagtaat 1740
aatatatttc ttcatgaaga cctcacagta aaaataggtg attttggtct agctacagtg 1800
aaatctcgat ggagtgggtc ccatcagttt gaacagttgt ctggatccat tttgtggatg 1860
gcaccagaag tcatcagaat gcaagataaa aatccataca gctttcagtc agatgtatat 1920
gcatttggaa ttgttctgta tgaattgatg actggacagt taccttattc aaacatcaac 1980
aacagggacc agataatttt tatggtggga cgaggatacc tgtctccaga tctcagtaag 2040
gtacggagta actgtccaaa agccatgaag agattaatgg cagagtgcct caaaaagaaa 2100
agagatgaga gaccactctt tccccaaatt ctcgcctcta ttgagctgct ggcccgctca 2160
ttgccaaaaa ttcaccgcag tgcatcagaa ccctccttga atcgggctgg tttccaaaca 2220
gaggatttta gtctatatgc ttgtgcttct ccaaaaacac ccatccaggc agggggatat 2280
ggtgcgtttc ctgtccactg a                                           2301
```

<210> 3
<211> 766
<212> PRT
<213> Homo sapiens

<400> 3

```
Met Ala Ala Leu Ser Gly Gly Gly Gly Gly Gly Ala Glu Pro Gly Gln
 1              5                   10                  15
Ala Leu Phe Asn Gly Asp Met Glu Pro Glu Ala Gly Ala Gly Ala Gly
            20                  25                  30
Ala Ala Ala Ser Ser Ala Ala Asp Pro Ala Ile Pro Glu Glu Val Trp
        35                  40                  45
Asn Ile Lys Gln Met Ile Lys Leu Thr Gln Glu His Ile Glu Ala Leu
    50                  55                  60
Leu Asp Lys Phe Gly Gly Glu His Asn Pro Pro Ser Ile Tyr Leu Glu
65                  70                  75                  80
Ala Tyr Glu Glu Tyr Thr Ser Lys Leu Asp Ala Leu Gln Gln Arg Glu
                85                  90                  95
Gln Gln Leu Leu Glu Ser Leu Gly Asn Gly Thr Asp Phe Ser Val Ser
            100                 105                 110
Ser Ser Ala Ser Met Asp Thr Val Thr Ser Ser Ser Ser Ser Ser Leu
        115                 120                 125
Ser Val Leu Pro Ser Ser Leu Ser Val Phe Gln Asn Pro Thr Asp Val
    130                 135                 140
Ala Arg Ser Asn Pro Lys Ser Pro Gln Lys Pro Ile Val Arg Val Phe
145                 150                 155                 160
Leu Pro Asn Lys Gln Arg Thr Val Val Pro Ala Arg Cys Gly Val Thr
                165                 170                 175
Val Arg Asp Ser Leu Lys Lys Ala Leu Met Met Arg Gly Leu Ile Pro
            180                 185                 190
Glu Cys Cys Ala Val Tyr Arg Ile Gln Asp Gly Glu Lys Lys Pro Ile
        195                 200                 205
Gly Trp Asp Thr Asp Ile Ser Trp Leu Thr Gly Glu Glu Leu His Val
    210                 215                 220
Glu Val Leu Glu Asn Val Pro Leu Thr Thr His Asn Phe Val Arg Lys
225                 230                 235                 240
Thr Phe Phe Thr Leu Ala Phe Cys Asp Phe Cys Arg Lys Leu Leu Phe
                245                 250                 255
Gln Gly Phe Arg Cys Gln Thr Cys Gly Tyr Lys Phe His Gln Arg Cys
            260                 265                 270
Ser Thr Glu Val Pro Leu Met Cys Val Asn Tyr Asp Gln Leu Asp Leu
        275                 280                 285
Leu Phe Val Ser Lys Phe Phe Glu His His Pro Ile Pro Gln Glu Glu
    290                 295                 300
Ala Ser Leu Ala Glu Thr Ala Leu Thr Ser Gly Ser Ser Pro Ser Ala
305                 310                 315                 320
Pro Ala Ser Asp Ser Ile Gly Pro Gln Ile Leu Thr Ser Pro Ser Pro
                325                 330                 335
Ser Lys Ser Ile Pro Ile Pro Gln Pro Phe Arg Pro Ala Asp Glu Asp
            340                 345                 350
His Arg Asn Gln Phe Gly Gln Arg Asp Arg Ser Ser Ser Ala Pro Asn
        355                 360                 365
Val His Ile Asn Thr Ile Glu Pro Val Asn Ile Asp Asp Leu Ile Arg
    370                 375                 380
Asp Gln Gly Phe Arg Gly Asp Gly Gly Ser Thr Thr Gly Leu Ser Ala
385                 390                 395                 400
Thr Pro Pro Ala Ser Leu Pro Gly Ser Leu Thr Asn Val Lys Ala Leu
                405                 410                 415
Gln Lys Ser Pro Gly Pro Gln Arg Glu Arg Lys Ser Ser Ser Ser Ser
            420                 425                 430
Glu Asp Arg Asn Arg Met Lys Thr Leu Gly Arg Arg Asp Ser Ser Asp
        435                 440                 445
Asp Trp Glu Ile Pro Asp Gly Gln Ile Thr Val Gly Gln Arg Ile Gly
    450                 455                 460
Ser Gly Ser Phe Gly Thr Val Tyr Lys Gly Lys Trp His Gly Asp Val
465                 470                 475                 480
Ala Val Lys Met Leu Asn Val Thr Ala Pro Thr Pro Gln Gln Leu Gln
                485                 490                 495
```

```
Ala Phe Lys Asn Glu Val Gly Val Leu Arg Lys Thr Arg His Val Asn
                500                 505                 510
Ile Leu Leu Phe Met Gly Tyr Ser Thr Lys Pro Gln Leu Ala Ile Val
            515                 520                 525
Thr Gln Trp Cys Glu Gly Ser Ser Leu Tyr His His Leu His Ile Ile
    530                 535                 540
Glu Thr Lys Phe Glu Met Ile Lys Leu Ile Asp Ile Ala Arg Gln Thr
545                 550                 555                 560
Ala Gln Gly Met Asp Tyr Leu His Ala Lys Ser Ile Ile His Arg Asp
                    565                 570                 575
Leu Lys Ser Asn Asn Ile Phe Leu His Glu Asp Leu Thr Val Lys Ile
                580                 585                 590
Gly Asp Phe Gly Leu Ala Thr Val Lys Ser Arg Trp Ser Gly Ser His
            595                 600                 605
Gln Phe Glu Gln Leu Ser Gly Ser Ile Leu Trp Met Ala Pro Glu Val
    610                 615                 620
Ile Arg Met Gln Asp Lys Asn Pro Tyr Ser Phe Gln Ser Asp Val Tyr
625                 630                 635                 640
Ala Phe Gly Ile Val Leu Tyr Glu Leu Met Thr Gly Gln Leu Pro Tyr
                    645                 650                 655
Ser Asn Ile Asn Asn Arg Asp Gln Ile Ile Phe Met Val Gly Arg Gly
                660                 665                 670
Tyr Leu Ser Pro Asp Leu Ser Lys Val Arg Ser Asn Cys Pro Lys Ala
            675                 680                 685
Met Lys Arg Leu Met Ala Glu Cys Leu Lys Lys Lys Arg Asp Glu Arg
    690                 695                 700
Pro Leu Phe Pro Gln Ile Leu Ala Ser Ile Glu Leu Leu Ala Arg Ser
705                 710                 715                 720
Leu Pro Lys Ile His Arg Ser Ala Ser Glu Pro Ser Leu Asn Arg Ala
                    725                 730                 735
Gly Phe Gln Thr Glu Asp Phe Ser Leu Tyr Ala Cys Ala Ser Pro Lys
                740                 745                 750
Thr Pro Ile Gln Ala Gly Gly Tyr Gly Ala Phe Pro Val His
            755                 760                 765
```

<210> 4
<211> 5765
<212> DNA
<213> Homo sapiens

<400> 4

```
tcctaggcgg cggccgcggc ggcggaggca gcagcggcgg cggcagtggc ggcggcgaag 60
gtggcggcgg ctcggccagt actcccggcc cccgccattt cggactggga gcgagcgcgg 120
cgcaggcact gaaggcggcg gcggggccag aggctcagcg gctcccaggt gcgggagaga 180
ggcctgctga aaatgactga atataaactt gtggtagttg gagctggtgg cgtaggcaag 240
agtgccttga cgatacagct aattcagaat cattttgtgg acgaatatga tccaacaata 300
gaggattcct acaggaagca agtagtaatt gatggagaaa cctgtctctt ggatattctc 360
gacacagcag gtcaagagga gtacagtgca atgagggacc agtacatgag gactggggag 420
ggctttcttt gtgtatttgc cataaataat actaaatcat ttgaagatat tcaccattat 480
agagaacaaa ttaaaagagt taaggactct gaagatgtac ctatggtcct agtaggaaat 540
aaatgtgatt tgccttctag aacagtagac acaaaacagg ctcaggactt agcaagaagt 600
tatggaattc cttttattga aacatcagca aagacaagac agggtgttga tgatgccttc 660
tatacattag ttcgagaaat tcgaaaacat aaagaaaaga tgagcaaaga tggtaaaaag 720
aagaaaaaga agtcaaagac aaagtgtgta attatgtaaa tacaatttgt actttttct 780
taaggcatac tagtacaagt ggtaattttt gtacattaca ctaaattatt agcatttgtt 840
ttagcattac ctaatttttt tcctgctcca tgcagactgt tagcttttac cttaaatgct 900
tattttaaaa tgacagtgga agttttttt tcctctaagt gccagtattc ccagagtttt 960
ggtttttgaa ctagcaatgc ctgtgaaaaa gaaactgaat acctaagatt tctgtcttgg 1020
ggtttttggt gcatgcagtt gattacttct tatttttctt accaattgtg aatgttggtg 1080
tgaaacaaat taatgaagct tttgaatcat ccctattctg tgttttatct agtcacataa 1140
atggattaat tactaatttc agttgagacc ttctaattgg tttttactga aacattgagg 1200
gaacacaaat ttatgggctt cctgatgatg attcttctag gcatcatgtc ctatagtttg 1260
tcatccctga tgaatgtaaa gttacactgt tcacaaaggt tttgtctcct ttccactgct 1320
```

attagtcatg gtcactctcc ccaaaatatt atattttttc tataaaaaga aaaaaatgga 1380
aaaaaattac aaggcaatgg aaactattat aaggccattt ccttttcaca ttagataaat 1440
tactataaag actcctaata gcttttcctg ttaaggcaga cccagtatga aatggggatt 1500
attatagcaa ccattttggg gctatattta catgctacta aatttttata ataattgaaa 1560
agattttaac aagtataaaa aattctcata ggaattaaat gtagtctccc tgtgtcagac 1620
tgctctttca tagtataact ttaaatcttt tcttcaactt gagtctttga agatagtttt 1680
aattctgctt gtgacattaa aagattattt gggccagtta tagcttatta ggtgttgaag 1740
agaccaaggt tgcaaggcca ggccctgtgt gaacctttga gctttcatag agagtttcac 1800
agcatggact gtgtccccac ggtcatccag tgttgtcatg cattggttag tcaaaatggg 1860
gagggactag ggcagtttgg atagctcaac aagatacaat ctcactctgt ggtggtcctg 1920
ctgacaaatc aagagcattg cttttgtttc ttaagaaaac aaactctttt ttaaaaatta 1980
cttttaaata ttaactcaaa agttgagatt ttggggtggt ggtgtgccaa gacattaatt 2040
ttttttttaa acaatgaagt gaaaaagttt tacaatctct aggtttggct agttctctta 2100
acactggtta aattaacatt gcataaacac ttttcaagtc tgatccatat ttaataatgc 2160
tttaaaataa aaataaaaac aatccttttg ataaatttaa aatgttactt attttaaaat 2220
aaatgaagtg agatggcatg gtgaggtgaa agtatcactg gactaggaag aaggtgactt 2280
aggttctaga taggtgtctt ttaggactct gattttgagg acatcactta ctatccattt 2340
cttcatgtta aaagaagtca tctcaaactc ttagtttttt tttttacaa ctatgtaatt 2400
tatattccat ttacataagg atacacttat ttgtcaagct cagcacaatc tgtaaatttt 2460
taacctatgt tacaccatct tcagtgccag tcttgggcaa aattgtgcaa gaggtgaagt 2520
ttatatttga atatccattc tcgttttagg actcttcttc catattagtg tcatcttgcc 2580
tccctacctt ccacatgccc catgacttga tgcagtttta atacttgtaa ttcccctaac 2640
cataagattt actgctgctg tggatatctc catgaagttt tcccactgag tcacatcaga 2700
aatgccctac atcttatttc ctcagggctc aagagaatct gacagatacc ataaagggat 2760
ttgacctaat cactaatttt caggtggtgg ctgatgcttt gaacatctct ttgctgccca 2820
atccattagc gacagtagga tttttcaaac ctggtatgaa tagacagaac cctatccagt 2880
ggaaggagaa tttaataaag atagtgctga aagaattcct taggtaatct ataactagga 2940
ctactcctgg taacagtaat acattccatt gttttagtaa ccagaaatct tcatgcaatg 3000
aaaaatactt taattcatga agcttacttt ttttttttgg tgtcagagtc tcgctcttgt 3060
cacccaggct ggaatgcagt ggcgccatct cagctcactg caacctccat ctcccaggtt 3120
caagcgattc tcgtgcctcg gcctcctgag tagctgggat tacaggcgtg tgccactaca 3180
ctcaactaat ttttgtattt ttaggagaga cggggtttca ccctgttggc caggctggtc 3240
tcgaactcct gacctcaagt gattcaccca ccttggcctc ataaacctgt tttgcagaac 3300
tcatttattc agcaaatatt tattgagtgc ctaccagatg ccagtcaccg cacaaggcac 3360
tgggtatatg gtatccccaa acaagagaca taatcccggt ccttaggtag tgctagtgtg 3420
gtctgtaata tcttactaag gcctttggta tacgacccag agataacacg atgcgtattt 3480
tagttttgca aagaaggggt ttggtctctg tgccagctct ataattgttt tgctacgatt 3540
ccactgaaac tcttcgatca agctacttta tgtaaatcac ttcattgttt taaaggaata 3600
aacttgatta tattgttttt ttatttggca taactgtgat tcttttagga caattactgt 3660
acacattaag gtgtatgtca gatattcata ttgacccaaa tgtgtaatat tccagttttc 3720
tctgcataag taattaaaat atacttaaaa attaatagtt ttatctgggt acaaataaac 3780
aggtgcctga actagttcac agacaaggaa acttctatgt aaaaatcact atgatttctg 3840
aattgctatg tgaaactaca gatctttgga acactgttta ggtagggtgt taagacttac 3900
acagtacctc gtttctacac agagaaagaa atggccatac ttcaggaact gcagtgctta 3960
tgaggggata tttaggcctc ttgaattttt gatgtagatg ggcatttttt taaggtagtg 4020
gttaattacc tttatgtgaa ctttgaatgg tttaacaaaa gatttgtttt tgtagagatt 4080
ttaaaggggg agaattctag aaataaatgt tacctaatta ttacagcctt aaagacaaaa 4140
atccttgttg aagttttttt aaaaaaagct aaattacata gacttaggca ttaacatgtt 4200
tgtggaagaa tatagcagac gtatattgta tcatttgagt gaatgttccc aagtaggcat 4260
tctaggctct atttaactga gtcacactgc ataggaattt agaacctaac ttttataggt 4320
tatcaaaact gttgtcacca ttgcacaatt ttgtcctaat atatacatag aaactttgtg 4380
gggcatgtta agttacagtt tgcacaagtt catctcattt gtattccatt gatttttttt 4440
ttcttctaaa catttttttct tcaaacagta tataactttt tttaggggat ttttttttag 4500
acagcaaaaa ctatctgaag atttccattt gtcaaaaagt aatgatttct tgataattgt 4560
gtagtaatgt tttttagaac ccagcagtta ccttaaagct gaatttatat ttagtaactt 4620
ctgtgttaat actggatagc atgaattctg cattgagaaa ctgaatagct gtcataaaat 4680
gaaactttct ttctaaagaa agatactcac atgagttctt gaagaatagt cataactaga 4740
ttaagatctg tgttttagtt taatagtttg aagtgcctgt ttgggataat gataggtaat 4800
ttagatgaat ttaggggaaa aaaaagttat ctgcagatat gttgagggcc catctctccc 4860
cccacacccc cacagagcta actgggttac agtgttttat ccgaaagttt ccaattccac 4920
tgtcttgtgt tttcatgttg aaaatacttt tgcatttttc ctttgagtgc caatttctta 4980
ctagtactat ttcttaatgt aacatgttta cctggaatgt attttaacta tttttgtata 5040
gtgtaaactg aaacatgcac attttgtaca ttgtgctttc ttttgtggga catatgcagt 5100

```
gtgatccagt tgttttccat catttggttg cgctgaccta ggaatgttgg tcatatcaaa 5160
cattaaaaat gaccactctt ttaattgaaa ttaactttta aatgtttata ggagtatgtg 5220
ctgtgaagtg atctaaaatt tgtaatattt ttgtcatgaa ctgtactact cctaattatt 5280
gtaatgtaat aaaaatagtt acagtgacta tgagtgtgta tttattcatg aaatttgaac 5340
tgtttgcccc gaaatggata tggaatactt tataagccat agacactata gtataccagt 5400
gaatctttta tgcagcttgt tagaagtatc ctttatttct aaaaggtgct gtggatatta 5460
tgtaaaggcg tgtttgctta aacttaaaac catatttaga agtagatgca aaacaaatct 5520
gcctttatga caaaaaaata ggataacatt atttatttat ttccttttat caaagaaggt 5580
aattgataca caacaggtga cttggtttta ggcccaaagg tagcagcagc aacattaata 5640
atggaaataa ttgaatagtt agttatgtat gttaatgcca gtcaccagca ggctatttca 5700
aggtcagaag taatgactcc atacatatta tttatttcta taactacatt taaatcatta 5760
ccagg                                                             5765
```

<210> 5
<211> 567
<212> DNA
<213> Homo sapiens

<400> 5

```
atgactgaat ataaacttgt ggtagttgga gctggtggcg taggcaagag tgccttgacg 60
atacagctaa ttcagaatca ttttgtggac gaatatgatc caacaataga ggattcctac 120
aggaagcaag tagtaattga tggagaaacc tgtctcttgg atattctcga cacagcaggt 180
caagaggagt acagtgcaat gagggaccag tacatgagga ctggggaggg ctttctttgt 240
gtatttgcca taaataatac taaatcattt gaagatattc accattatag agaacaaatt 300
aaaagagtta aggactctga agatgtacct atggtcctag taggaaataa atgtgatttg 360
ccttctagaa cagtagacac aaaacaggct caggacttag caagaagtta tggaattcct 420
tttattgaaa catcagcaaa gacaagacag ggtgttgatg atgccttcta tacattagtt 480
cgagaaattc gaaaacataa agaaaagatg agcaaagatg gtaaaaagaa gaaaaagaag 540
tcaaagacaa agtgtgtaat tatgtaa                                     567
```

<210> 6
<211> 188
<212> PRT
<213> Homo sapiens

<400> 6

```
Met Thr Glu Tyr Lys Leu Val Val Val Gly Ala Gly Gly Val Gly Lys
 1               5                  10                  15
Ser Ala Leu Thr Ile Gln Leu Ile Gln Asn His Phe Val Asp Glu Tyr
            20                  25                  30
Asp Pro Thr Ile Glu Asp Ser Tyr Arg Lys Gln Val Val Ile Asp Gly
        35                  40                  45
Glu Thr Cys Leu Leu Asp Ile Leu Asp Thr Ala Gly Gln Glu Glu Tyr
    50                  55                  60
Ser Ala Met Arg Asp Gln Tyr Met Arg Thr Gly Glu Gly Phe Leu Cys
65                  70                  75                  80
Val Phe Ala Ile Asn Asn Thr Lys Ser Phe Glu Asp Ile His His Tyr
                85                  90                  95
Arg Glu Gln Ile Lys Arg Val Lys Asp Ser Glu Asp Val Pro Met Val
            100                 105                 110
Leu Val Gly Asn Lys Cys Asp Leu Pro Ser Arg Thr Val Asp Thr Lys
        115                 120                 125
Gln Ala Gln Asp Leu Ala Arg Ser Tyr Gly Ile Pro Phe Ile Glu Thr
    130                 135                 140
Ser Ala Lys Thr Arg Gln Gly Val Asp Asp Ala Phe Tyr Thr Leu Val
145                 150                 155                 160
Arg Glu Ile Arg Lys His Lys Glu Lys Met Ser Lys Asp Gly Lys Lys
                165                 170                 175
Lys Lys Lys Lys Ser Lys Thr Lys Cys Val Ile Met
            180                 185
```

<210> 7
<211> 4454
<212> DNA
<213> Homo sapiens

<400> 7

gaaacgtccc gtgtgggagg ggcgggtctg ggtgcggcct gccgcatgac tcgtggttcg 60
gaggcccacg tggccggggc ggggactcag gcgcctgggg cgccgactga ttacgtagcg 120
ggcggggccg gaagtgccgc tccttggtgg gggctgttca tggcggttcc ggggtctcca 180
acatttttcc cggctgtggt cctaaatctg tccaaagcag aggcagtgga gcttgaggtt 240
cttgctggtg tgaaatgact gagtacaaac tggtggtggt tggagcaggt ggtgttggga 300
aaagcgcact gacaatccag ctaatccaga accactttgt agatgaatat gatcccacca 360
tagaggattc ttacagaaaa caagtggtta tagatggtga aacctgtttg ttggacatac 420
tggatacagc tggacaagaa gagtacagtg ccatgagaga ccaatacatg aggacaggcg 480
aaggcttcct ctgtgtattt gccatcaata atagcaagtc atttgcggat attaacctct 540
acagggagca gattaagcga gtaaaagact cggatgatgt acctatggtg ctagtgggaa 600
acaagtgtga tttgccaaca aggacagttg atacaaaaca agcccacgaa ctggccaaga 660
gttacgggat tccattcatt gaaacctcag ccaagaccag acagggtgtt gaagatgctt 720
tttacacact ggtaagagaa atacgccagt accgaatgaa aaaactcaac agcagtgatg 780
atgggactca gggttgtatg ggattgccat gtgtggtgat gtaacaagat acttttaaag 840
ttttgtcaga aaagagccac tttcaagctg cactgacacc ctggtcctga cttccctgga 900
ggagaagtat tcctgttgct gtcttcagtc tcacagagaa gctcctgcta cttccccagc 960
tctcagtagt ttagtacaat aatctctatt tgagaagttc tcagaataac tacctcctca 1020
cttggctgtc tgaccagaga atgcacctct tgttactccc tgttattttt ctgccctggg 1080
ttcttccaca gcacaaacac acctctgcca ccccaggttt ttcatctgaa aagcagttca 1140
tgtctgaaac agagaaccaa accgcaaacg tgaaattcta ttgaaaacag tgtcttgagc 1200
tctaaagtag caactgctgg tgattttttt tttcttttta ctgttgaact tagaactatg 1260
ctaatttttg gagaaatgtc ataaattact gttttgccaa gaatatagtt attattgctg 1320
tttggtttgt ttataatgtt atcggctcta ttctctaaac tggcatctgc tctagattca 1380
taaatacaaa aatgaatact gaattttgag tctatcctag tcttcacaac tttgacgtaa 1440
ttaaatccaa ctttcacagt gaagtgcctt tttcctagaa gtggtttgta gacttccttt 1500
ataatatttc agtggaatag atgtctcaaa aatccttatg catgaaatga atgtctgaga 1560
tacgtctgtg acttatctac cattgaagga aagctatatc tatttgagag cagatgccat 1620
tttgtacatg tatgaaattg gttttccaga ggcctgtttt ggggctttcc caggagaaag 1680
atgaaactga aagcacatga ataatttcac ttaataattt ttacctaatc tccacttttt 1740
tcataggtta ctacctatac aatgtatgta atttgtttcc cctagcttac tgataaacct 1800
aatattcaat gaacttccat ttgtattcaa atttgtgtca taccagaaag ctctacattt 1860
gcagatgttc aaatattgta aaactttggt gcattgttat ttaatagctg tgatcagtga 1920
ttttcaaacc tcaaatatag tatattaaca aattacattt tcactgtata tcatggtatc 1980
ttaatgatgt atataattgc cttcaatccc cttctcaccc caccctctac agcttccccc 2040
acagcaatag gggcttgatt atttcagttg agtaaagcat ggtgctaatg gaccagggtc 2100
acagtttcaa aacttgaaca atccagttag catcacagag aaagaaattc ttctgcattt 2160
gctcattgca ccagtaactc cagctagtaa ttttgctagg tagctgcagt tagccctgca 2220
aggaaagaag aggtcagtta gcacaaaccc tttaccatga ctggaaaact cagtatcacg 2280
tatttaaaca tttttttttc ttttagccat gtagaaactc taaattaagc caatattctc 2340
atttgagaat gaggatgtct cagctgagaa acgttttaaa ttctctttat tcataatgtt 2400
ctttgaaggg tttaaaacaa gatgttgata aatctaagct gatgagtttg ctcaaaacag 2460
gaagttgaaa ttgttgagac aggaatggaa aatataatta attgatacct atgaggattt 2520
ggaggcttgg cattttaatt tgcagataat accctggtaa ttctcatgaa aaatagactt 2580
ggataacttt tgataaaaga ctaattccaa aatggccact ttgttcctgt ctttaatatc 2640
taaatactta ctgaggtcct ccatcttcta tattatgaat tttcatttat taagcaaatg 2700
tcatattacc ttgaaattca gaagagaaga aacatatact gtgtccagag tataatgaac 2760
ctgcagagtt gtgcttctta ctgctaattc tgggagcttt cacagtactg tcatcatttg 2820
taaatggaaa ttctgctttt ctgtttctgc tccttctgga gcagtgctac tctgtaattt 2880
tcctgaggct tatcacctca gtcatttctt ttttaaatgt ctgtgactgg cagtgattct 2940
ttttcttaaa aatctattaa atttgatgtc aaattaggga gaaagatagt tactcatctt 3000
gggctcttgt gccaatagcc cttgtatgta tgtacttaga gttttccaag tatgttctaa 3060
gcacagaagt ttctaaatgg ggccaaaatt cagacttgag tatgttcttt gaatacctta 3120
agaagttaca attagccggg catggtggcc cgtgcctgta gtcccagcta cttgagaggc 3180
tgaggcagga gaatcacttc aacccaggag gtggaggtta cagtgagcag agatcgtgcc 3240
actgcactcc agcctgggtg acaagagaga cttgtctcca aaaaaaaagt tacacctagg 3300
tgtgaatttt ggcacaaagg agtgacaaac ttatagttaa aagctgaata acttcagtgt 3360
ggtataaaac gtggttttta ggctatgttt gtgattgctg aaaagaattc tagtttacct 3420
caaaatcctt ctctttcccc aaattaagtg cctggccagc tgtcataaat tacatattcc 3480
ttttggtttt tttaaaggtt acatgttcaa gagtgaaaat aagatgttct gtctgaaggc 3540

```
taccatgccg gatctgtaaa tgaacctgtt aaatgctgta tttgctccaa cggcttacta 3600
tagaatgtta cttaatacaa tatcatactt attacaattt ttactatagg agtgtaatag 3660
gtaaaattaa tctctatttt agtgggccca tgtttagtct ttcaccatcc tttaaactgc 3720
tgtgaatttt tttgtcatga cttgaaagca aggatagaga aacactttag agatatgtgg 3780
ggttttttta ccattccaga gcttgtgagc ataatcatat ttgctttata tttatagtca 3840
tgaactccta agttggcagc tacaaccaag aaccaaaaaa tggtgcgttc tgcttcttgt 3900
aattcatctc tgctaataaa ttataagaag caaggaaaat tagggaaaat attttatttg 3960
gatggtttct ataaacaagg gactataatt cttgtacatt atttttcatc tttgctgttt 4020
ctttgagcag tctaatgtgc cacacaatta tctaaggtat ttgttttcta taagaattgt 4080
tttaaaagta ttcttgttac cagagtagtt gtattatatt tcaaaacgta agatgatttt 4140
taaaagcctg agtactgacc taagatggaa ttgtatgaac tctgctctgg agggaggga 4200
ggatgtccgt ggaagttgta agacttttat ttttttgtgc catcaaatat aggtaaaaat 4260
aattgtgcaa ttctgctgtt taaacaggaa ctattggcct ccttggccct aaatggaagg 4320
gccgatattt taagttgatt attttattgt aaattaatcc aacctagttc tttttaattt 4380
ggttgaatgt tttttcttgt taaatgatgt ttaaaaaata aaaactggaa gttcttggct 4440
tagtcataat tctt                                                   4454
```

<210> 8
<211> 570
<212> DNA
<213> Homo sapiens

<400> 8

```
atgactgagt acaaactggt ggtggttgga gcaggtggtg ttgggaaaag cgcactgaca 60
atccagctaa tccagaacca ctttgtagat gaatatgatc ccaccataga ggattcttac 120
agaaaacaag tggttataga tggtgaaacc tgtttgttgg acatactgga tacagctgga 180
caagaagagt acagtgccat gagagaccaa tacatgagga caggcgaagg cttcctctgt 240
gtatttgcca tcaataatag caagtcattt gcggatatta acctctacag ggagcagatt 300
aagcgagtaa aagactcgga tgatgtacct atggtgctag tgggaaacaa gtgtgatttg 360
ccaacaagga cagttgatac aaaacaagcc cacgaactgg ccaagagtta cgggattcca 420
ttcattgaaa cctcagccaa gaccagacag ggtgttgaag atgcttttta cacactggta 480
agagaaatac gccagtaccg aatgaaaaaa ctcaacagca gtgatgatgg gactcagggt 540
tgtatgggat tgccatgtgt ggtgatgtaa                                  570
```

<210> 9
<211> 189
<212> PRT
<213> Homo sapiens

<400> 9

```
Met Thr Glu Tyr Lys Leu Val Val Val Gly Ala Gly Gly Val Gly Lys
 1               5                  10                  15
Ser Ala Leu Thr Ile Gln Leu Ile Gln Asn His Phe Val Asp Glu Tyr
            20                  25                  30
Asp Pro Thr Ile Glu Asp Ser Tyr Arg Lys Gln Val Val Ile Asp Gly
        35                  40                  45
Glu Thr Cys Leu Leu Asp Ile Leu Asp Thr Ala Gly Gln Glu Glu Tyr
    50                  55                  60
Ser Ala Met Arg Asp Gln Tyr Met Arg Thr Gly Glu Gly Phe Leu Cys
65                  70                  75                  80
Val Phe Ala Ile Asn Asn Ser Lys Ser Phe Ala Asp Ile Asn Leu Tyr
                85                  90                  95
Arg Glu Gln Ile Lys Arg Val Lys Asp Ser Asp Asp Val Pro Met Val
            100                 105                 110
Leu Val Gly Asn Lys Cys Asp Leu Pro Thr Arg Thr Val Asp Thr Lys
        115                 120                 125
Gln Ala His Glu Leu Ala Lys Ser Tyr Gly Ile Pro Phe Ile Glu Thr
    130                 135                 140
Ser Ala Lys Thr Arg Gln Gly Val Glu Asp Ala Phe Tyr Thr Leu Val
145                 150                 155                 160
Arg Glu Ile Arg Gln Tyr Arg Met Lys Lys Leu Asn Ser Ser Asp Asp
                165                 170                 175
Gly Thr Gln Gly Cys Met Gly Leu Pro Cys Val Val Met


                    180                 185
```